# EUROPEAN PATENT APPLICATION

(11) **EP 4 806 382 A1**
(43) Date of publication of application: **16.09.2026**
(21) Application number: 26163468.7
(22) Date of filing: 10.03.2026
(51) Int. Cl.: A61B 46/10, A61B 34/30, A61M 25/01

(54) **DRAPING SYSTEM FOR ROBOTIC MEDICAL PROCEDURE SYSTEMS**

(30) Priority: 11.03.2025 US 202519075848
(71) Applicant: Siemens Healthineers Endovascular Robotics, Inc., Newton, MA 02466 (US)
(72) Inventor: HUANG, Zilong, Newton, MA, 02466 (US); FALB, Peter, Newton, MA, 02466 (US); BRAUER, Jacob, Newton, MA, 02466 (US); KASVIKIS, Dino, 02452 Waltham, MA (US)
(74) Representative: HKW Intellectual Property PartG mbB

(57) **Abstract**

A draping system for a robotic drive having an elongated drive body and an articulating support arm supporting the elongated drive body, the draping system comprising: a first tubular drape configured to cover a first portion of the elongated drive body; a second tubular drape configured to cover a second portion of the elongated drive body, the second tubular drape being separate from the first tubular drape; and a third drape configured to cover the articulating support arm, the third drape being separate from the first tubular drape and the second tubular drape.

## Description

### FIELD

One or more example embodiments relate generally to sterile draping systems for robotic medical procedure systems and/or methods for draping robotic medical procedure systems.

### BACKGROUND

Catheters, guidewires and other elongated medical devices (EMDs) may be used in minimally-invasive medical procedures for diagnosing and/or treating diseases of various vascular systems. Example medical procedures include neurovascular intervention (NVI), percutaneous coronary intervention (PCI) and peripheral vascular intervention (PVI). These procedures typically involve navigating a guidewire through the vasculature to advance a catheter to deliver therapy. Robotic catheter-based procedure systems may be used to aid a physician in performing medical procedures such as those mentioned above.

### SUMMARY

One or more example embodiments provide a draping system for draping a (e.g., T-shaped) robotic medical procedure system for use in a medical procedure. The robotic medical procedure system includes, among other things, a drive body and a positioning system (also referred to herein as a support arm). According to at least one example embodiment, the draping system is configured such that a channel located at an under side of the drive body, which allows linear modules to move during use, remains exposed after draping. The draping system also allows for a consumable device to be attached to the drive body after draping. In at least one example embodiment, the draping system is attached relatively firmly to help avoid being caught and/or torn by the consumable device. The draping system may be deployed relatively quickly (e.g., in about three minutes).

According to at least one example embodiment, the draping system includes three separate (individual) drapes that cover the robotic medical procedure system from the left, the right, and the bottom. The left (distal) drape and the right (proximal) drape are tube or tubular shaped to slide over the left (distal) and right (proximal) ends of the drive body, whereas the bottom (arm) drape is a flatter drape that is wrapped upward around the support arm. Perforated tape may be used to maintain the tube shape of the distal drape and the proximal drape temporarily when draping the drive body. After the drive body is draped with a respective one of the distal and proximal drape, the perforated tape is torn off to expose the channel under the drive body. The draping system also includes front plates and bottom plates with fasteners (e.g., magnets, snaps, etc.) that may keep the drapes away from the movable device modules. The arm drape is clipped onto the support arm below the drive body and then wrapped upward around the portions of the support arm more distal from the drive body.

At least one example embodiment provides a draping system for a robotic drive having an elongated drive body and an articulating support arm supporting the elongated drive body, the draping system comprising: a first tubular drape configured to cover a first portion of the elongated drive body; a second tubular drape configured to cover a second portion of the elongated drive body, the second tubular drape being separate from the first tubular drape; and a third drape configured to cover the articulating support arm, the third drape being separate from the first tubular drape and the second tubular drape.

At least one other example embodiment provides a sterile barrier for a robotic drive having an elongated drive body and an articulating support arm supporting the elongated drive body, the sterile barrier comprising: a first tubular drape having a first rigid plate, a second rigid plate and a first perforated tape extending along a length of the first tubular drape, the first perforated tape being temporarily adhered to the first rigid plate and the second rigid plate to maintain a tubular shape of the first tubular drape, and the first tubular drape configured to cover a first portion of the elongated drive body when deployed.

According to one or more example embodiments, the first tubular drape may be configured to slide onto the elongated drive body from a first end of the elongated drive body to cover the first portion of the elongated drive body. The second tubular drape may be configured to slide onto the elongated drive body from a second end of the elongated drive body to cover the second portion of the elongated drive body, the second end being opposite the first end in a longitudinal direction of the elongated drive body.

The first tubular drape may include a first perforated tape temporarily adhered along a length of the first tubular drape to maintain a tubular shape of the first tubular drape.

The first perforated tape may be configured to be removed after the first tubular drape is deployed on the elongated drive body.

The robotic drive may include one or more movable drive modules configured to move along a length of the elongated drive body via a channel formed along a length of a bottom wall of the elongated drive body, and removal of the first perforated tape may expose the channel.

The first tubular drape may include a first rigid plate and a second rigid plate extending along a length of the first tubular drape. The first perforated tape may be temporarily adhered to the first rigid plate and the second rigid plate to maintain the tubular shape of the first tubular drape.

The first tubular drape may include a flexible drape body, and the first rigid plate and the second rigid plate may be adhered to respective edges of the flexible drape body.

The second tubular drape may have a third rigid plate, a fourth rigid plate and a second perforated tape extending along a length of the second tubular drape. The second perforated tape may be temporarily adhered to the third rigid plate and the fourth rigid plate to maintain a tubular shape of the second tubular drape.

The second tubular drape may include a flexible drape body, and the third rigid plate and the fourth rigid plate may be adhered to respective edges of the flexible drape body.

Third drape may be a non-tubular drape.

The third drape may include a c-shaped clip configured to be removably secured to a portion of an arm supporting the elongated drive body, and a lower arm drape portion configured to wrap around the support arm.

At least one other example embodiment provides a method for draping a robotic drive having an elongated drive body and an articulating support arm supporting the elongated drive body, the method comprising: sliding a first tubular drape onto a first portion of the elongated drive body; sliding a second tubular drape onto a second portion of the elongated drive body, the second tubular drape being separate from the first tubular drape; and covering the articulating support arm with a third drape, the third drape being separate from the first tubular drape and the second tubular drape.

At least one of (i) the sliding of the first tubular drape onto the first portion may include sliding the first tubular drape onto the first portion from a first longitudinal end of the elongated drive body; or (ii) the sliding of the second tubular drape onto the second portion may include sliding the second tubular drape onto the second portion from a second longitudinal end of the elongated drive body. The second longitudinal end may be opposite the first longitudinal end.

The robotic drive may include one or more movable drive modules configured to move along a length of the elongated drive body via a channel formed along a length of a bottom wall of the elongated drive body. The method may further include: moving the one or more movable drive modules to the second longitudinal end of the elongated drive body prior to sliding the first tubular drape onto the first portion, and moving the one or more movable drive modules to the first longitudinal end of the elongated drive body prior to sliding the second tubular drape onto the second portion.

Prior to moving the one or more movable drive modules to the first longitudinal end, the method may further include removing a first perforated tape from the first tubular drape to expose at least a portion of the channel.

The first tubular drape may include a first rigid plate and a second rigid plate extending along a length of the first tubular drape, and the method may include removably fixing the first rigid plate to a first side of the elongated drive body, and removably fixing the second rigid plate to a second side of the elongated drive body, the second side being adjacent the first side.

The second tubular drape may have a third rigid plate and a fourth rigid plate extending along a length of the second tubular drape, and the method may include removably fixing the third rigid plate to the first side of the elongated drive body, and removably fixing the fourth rigid plate to the second side of the elongated drive body.

Third drape may include a c-shaped clip and a lower arm drape portion, and the covering may include removably securing the c-shaped clip to a portion of the articulating support arm, and wrapping the lower arm drape portion around the articulating support arm.

The scope of protection sought for various example embodiments is set out by the independent claims. The example embodiments and/or features, if any, described in this specification that do not fall under the scope of the independent claims are to be interpreted as examples useful for understanding various embodiments.

Independent of the grammatical term usage, individuals with male, female or other gender identities are included within the term.

### BRIEF DESCRIPTION OF THE DRAWINGS

Example embodiments will become more fully understood from the detailed description given herein below and the accompanying drawings, wherein like elements are represented by like reference numerals, which are given by way of illustration only and thus are not limiting of this disclosure.
FIG. 1 is a perspective view of a portion of an example catheter-based procedure system in accordance with example embodiments.
FIG. 2 is a view from the proximal end of the robotic drive of the catheter-based procedure system shown in FIG. 1.
FIG. 3 is an inside plan view of a distal drape according to example embodiments.
FIG. 4 is a front perspective view of the distal drape shown in FIG. 3.
FIG. 5 is an exploded view of the distal rigid front plate, the distal rigid bottom plate and the distal perforated tape of the distal drape shown in FIGS. 3 and 4.
FIG. 6 is a rear perspective view of a portion of the catheter-based procedure system having the distal drape shown in FIGS. 3 and 4 deployed thereon.
FIG. 7 is a view from the distal end of the catheter-based procedure system having the distal drape shown in FIGS. 3 and 4 deployed thereon.
FIG. 8 is an inside plan view of a proximal drape according to example embodiments.
FIG. 9 is a front perspective view of the proximal drape shown in FIG. 8.
FIG. 10 is an exploded view of the proximal rigid front plate, the proximal rigid bottom plate and the proximal perforated tape of the proximal drape shown in FIGS. 8 and 9.
FIG. 11 is a rear perspective view of a portion of the catheter-based procedure system having the proximal drape shown in FIGS. 8 and 9 applied thereto.
FIG. 12 is an inside plan view of an arm drape according to example embodiments.
FIG. 13 is a perspective view of the arm drape shown in FIG. 12.
FIG. 14 is a view of the arm drape partially attached to the positioning system of the catheter-based procedure system.
FIG. 15 is a cross-sectional view of an example embodiment of the rigid front plate and the rigid bottom plate deployed on the robotic drive.
FIG. 16 is a cross-sectional view of another example embodiment of the rigid front plate and the rigid bottom plate deployed on the robotic drive.
FIG. 17 is a perspective view of an example embodiment of the draping system deployed on the catheter-based procedure system 10.
FIG. 18 illustrates a drive module moving through a channel on the bottom wall of the robotic drive.
FIGS. 19A-19K illustrate a method for deploying, on a catheter-based procedure system, the draping system described herein with regard to one or more example embodiments.
FIG. 20 is a view of a first longitudinal wall side of a catheter-based procedure system draped with a draping system according to one or more example embodiments.
It should be noted that these figures are intended to illustrate the general characteristics of methods, structure and/or materials utilized in certain example embodiments and to supplement the written description provided below. These drawings are not, however, to scale and may not precisely reflect the precise structural or performance characteristics of any given embodiment and should not be interpreted as defining or limiting the range of values or properties encompassed by example embodiments. The use of similar or identical reference numbers in the various drawings is intended to indicate the presence of a similar or identical element or feature.

### DETAILED DESCRIPTION

Various example embodiments will now be described more fully with reference to the accompanying drawings in which some example embodiments are shown.

Detailed illustrative embodiments are disclosed herein. However, specific structural and functional details disclosed herein are merely representative for purposes of describing example embodiments. The example embodiments may, however, be embodied in many alternate forms and should not be construed as limited to only the embodiments set forth herein.

It should be understood that there is no intent to limit example embodiments to the particular forms disclosed. On the contrary, example embodiments are to cover all modifications, equivalents, and alternatives falling within the scope of this disclosure. Like numbers refer to like elements throughout the description of the figures.

As discussed herein the terminology "one or more" and "at least one" may be used interchangeably.

It will be appreciated that a number of example embodiments may be used in combination.

Unless specifically stated otherwise as apparent from the following discussion, it is appreciated that throughout the present disclosure, discussions utilizing terms such as "accessing," "determining," "storing," "assigning," "adjusting," "combining," "summing," "adding," "optimizing," "minimizing," producing," "generating," "identifying," "setting," "increasing," "evaluating," "calculating," or the like, may refer to actions and processes of a controller, computer system or similar electronic computing device or processor. The controller, computer system or similar electronic computing device manipulates and transforms data represented as physical (electronic) quantities within the computer system memories, registers or other such information storage, transmission or display devices.

One or more example embodiments will be discussed herein with regard to robotic catheter-based procedure systems, catheters (or microcatheters) and guidewires. However, example embodiments should not be limited the examples discussed herein. Rather, one or more example embodiments may be applicable to other robotic medical device or procedure systems.

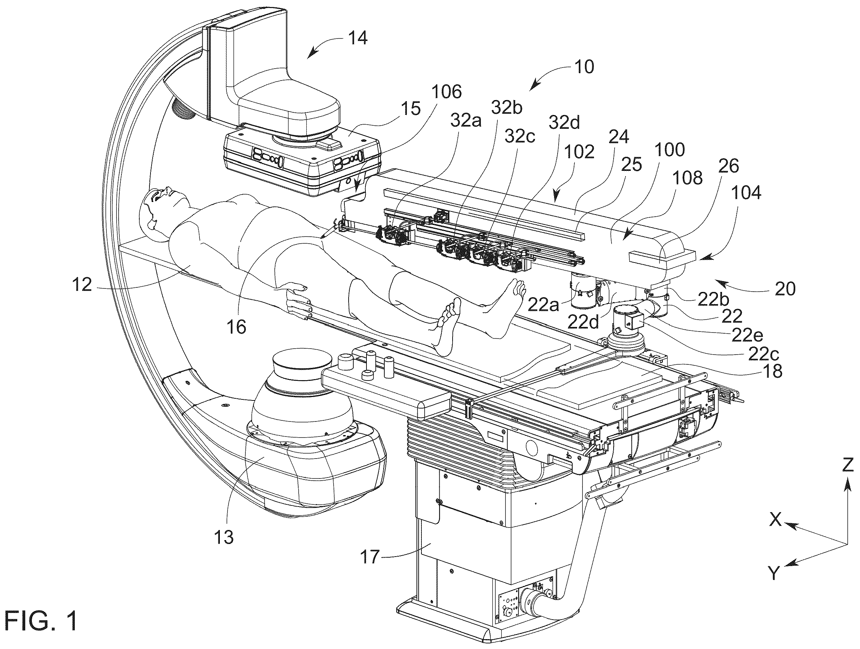

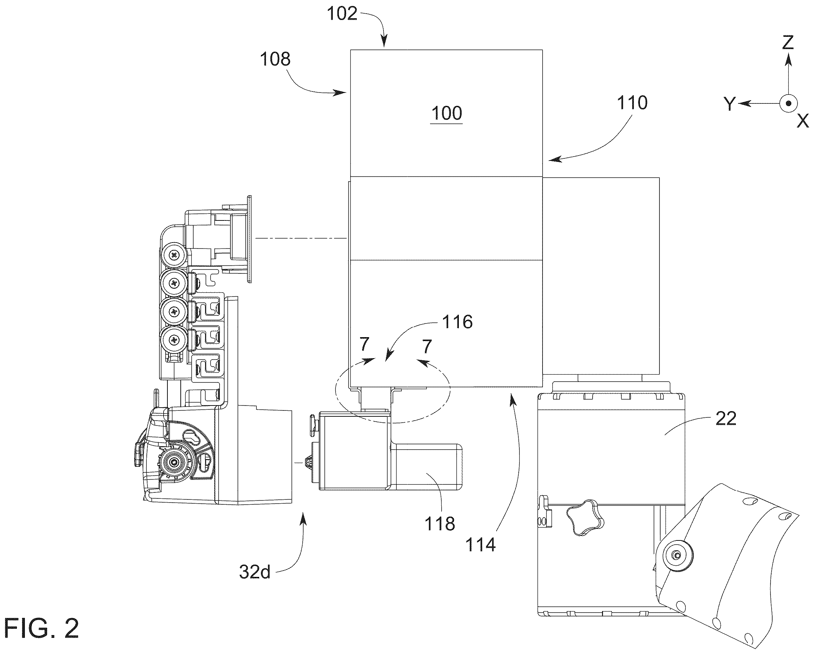

FIG. 1 is a perspective view of an example catheter-based procedure system 10 in accordance with an example embodiment. FIG. 2 is a view from the proximal end of the robotic drive of the catheter-based procedure system 10.

Catheter-based procedure system 10 may be used to perform catheter-based medical procedures including, for example, percutaneous intervention procedures such as a percutaneous coronary intervention (PCI), a neurovascular interventional procedure (NVI) and/or peripheral vascular intervention procedures (PVI).

Catheter-based medical procedures may include diagnostic catheterization procedures during which one or more catheters or other EMDs are used to aid in the diagnosis of a patient's disease. For example, during a catheter-based diagnostic procedure, a contrast media may be injected into one or more arteries through a catheter and an image of the patient's vasculature, including various vascular networks, is taken.

Catheter-based medical procedures may also include catheter-based therapeutic procedures (e.g., angioplasty, stent placement, treatment of peripheral vascular disease, clot removal, arterial venous malformation therapy, treatment of aneurysm, etc.) during which a catheter (or other EMD) is used to treat a disease.

Referring now to FIGS. 1 and 2, catheter-based procedure system 10 includes, among other elements, a bedside unit 20 and a control station (not shown). Bedside unit 20 includes a robotic drive 24 and a positioning system 22 that are located adjacent to a patient 12. Patient 12 is supported on a patient table 18. The positioning system 22 is used to position and support the robotic drive 24. In the example embodiment shown in FIG. 1, the positioning system 22 includes an articulating arm, which further includes rotational joints 22a, 22b and 22c and arms 22e and 22d extending between the rotational joints. The rotational joint 22c is arranged on an attachment mechanism that attaches to the patient table 18 (as shown in FIG. 1), a base, or a cart. The rotational joint 22a is attached to the robotic drive 24. The positioning system 22 may be moved out of the way (along with the robotic drive 24) to allow for the patient 12 to be placed on the patient table 18. Once the patient 12 is positioned on the patient table 18, the positioning system 22 may be used to situate or position the robotic drive 24 relative to the patient 12 for the procedure.

Patient table 18 is operably supported by a pedestal 17, which is secured to the floor and/or earth. Patient table 18 is able to move with multiple degrees of freedom, for example, roll, pitch, and yaw, relative to the pedestal 17.

Generally, the robotic drive 24 may be equipped with the appropriate percutaneous interventional devices and accessories (e.g., guidewires, various types of catheters including balloon catheters, stent delivery systems, stent retrievers, embolization coils, liquid embolics, aspiration pumps, device to deliver contrast media, medicine, hemostasis valve adapters, syringes, stopcocks, inflation device, etc.) to allow a user or operator to perform a catheter-based medical procedure via a robotic system by operating various controls such as the controls and inputs located at the control station. The robotic drive 24 includes a plurality of device modules 32a-32d mounted to a rail or linear member. Each of the device modules 32a-32d may be used to drive an EMD such as a catheter or guidewire. For example, the robotic drive 24 may be used to automatically feed a guidewire into a diagnostic catheter and into a guide catheter in an artery of the patient 12. One or more devices, such as an EMD, enter the body (e.g., a vessel) of the patient 12 at an insertion point 16 via, for example, an introducer sheath.

Catheter-based procedure system 10 also includes an imaging system 14. Imaging system 14 may be any medical imaging system that may be used in conjunction with a catheter based medical procedure (e.g., non-digital X-ray, digital X-ray, CT, MRI, ultrasound, etc.). In an example embodiment, imaging system 14 is a digital X-ray imaging device that is in communication with the control station. In one embodiment, imaging system 14 may include a C-arm that allows imaging system 14 to partially or completely rotate around patient 12 in order to obtain images at different angular positions relative to patient 12 (e.g., sagittal views, caudal views, anterior-posterior views, etc.). In one example embodiment, imaging system 14 may be a fluoroscopy system including a C-arm having an X-ray source 13 and a detector 15, also known as an image intensifier.

Imaging system 14 may be configured to take X-ray images of the appropriate area of patient 12 during a procedure. For example, imaging system 14 may be configured to take one or more X-ray images of the head to diagnose a neurovascular condition. Imaging system 14 may also be configured to take one or more X-ray images (e.g., real time images) during a catheter-based medical procedure to assist the user or operator to properly position a guidewire, guide catheter, microcatheter, stent retriever, coil, stent, balloon, etc. during the procedure. The image or images may be displayed on a display. For example, images may be displayed on a display to allow the user or operator to accurately move a guide catheter or guidewire into the proper position.

In order to clarify directions, a rectangular coordinate system is introduced with X, Y, and Z axes. The positive X axis is oriented in a longitudinal (axial) distal direction, that is, in the direction from the proximal end to the distal end, stated another way from the proximal to distal direction. The Y and Z axes are in a transverse plane to the X axis, with the positive Z axis oriented up, that is, in the direction opposite of gravity, and the Y axis is automatically determined by right-hand rule.

Still referring to FIGS. 1 and 2, robotic drive 24 includes a drive body 100 (also referred to as an elongated drive body) having a top wall 102, a proximal wall 104, a distal wall 106, a first longitudinal wall 108, and a second longitudinal wall 110 opposite the first longitudinal wall 108. When robotic drive 24 is in an in-use position on patient table 18, top wall 102 is the furthest wall from patient table 18, proximal wall 104 is the wall most proximal or closest to a foot of patient table 18. Distal wall 106 is the wall most distal or closest to the head of patient table 18. The first longitudinal wall 108 and the second longitudinal wall 110 are the walls closest to and furthest from a bed rail closest the base (support arm) of positioning system 22. Drive body 100 further includes a bottom wall 114 that is closest to patient table 18 when robotic drive 24 is in the in-use position. Bottom wall 114 has a slot 116 extending longitudinally between proximal wall 104 and distal wall 106.

A distal handle 25 may be arranged or formed on the first longitudinal wall 108 as shown in FIG. 1. A proximal handle 26 may be arranged or formed at a proximal end of the drive body 100. As shown in FIG. 1, the proximal handle 26 may extend from the first longitudinal wall 108 to the second longitudinal wall 110 passing around the proximal wall 104.

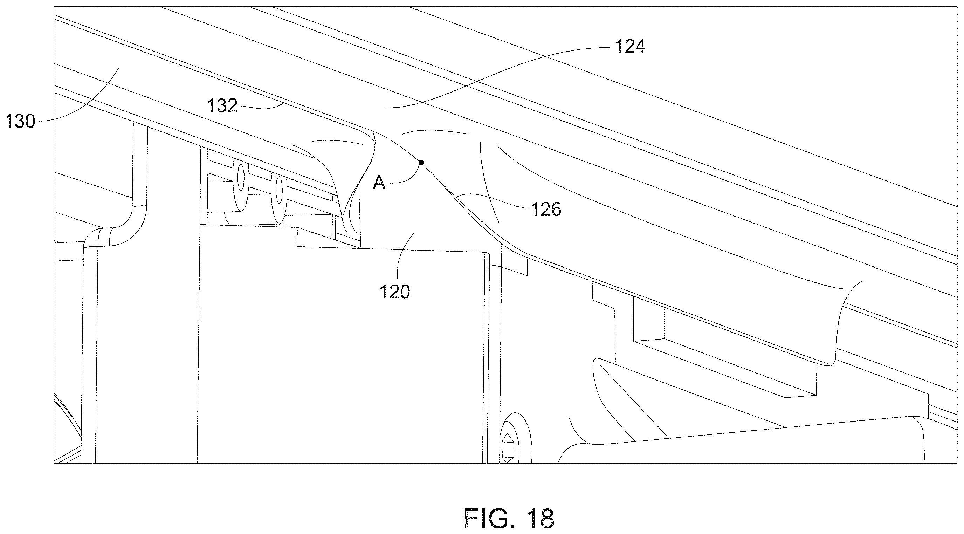

As shown in FIG. 2, device module 32d includes a drive module 118 that is driven along a longitudinal axis of drive body 100. Drive module 118 includes a connecting stage member 120 (FIG. 18) extending through slot 116 and is operatively engaged with a drive mechanism that moves drive module 118 along the longitudinal axis of drive body 100.

Prior to performing a medical procedure using the catheter-based procedure system 10, the system should be draped to provide a sterile barrier between the patient 12 and the non-sterile portions of the system. One or more example embodiments provide a draping system for draping the catheter-based procedure system 10 for use in a medical procedure. According to one or more example embodiments, the draping system is configured such that a channel located at an under side of the drive body 100, which allows the drive modules 32a-32d to move during use, remains exposed after draping. The draping system, according to one or more example embodiments, also allows for a consumable device to be attached to the drive body 100 after draping. An example of the consumable device is described in U.S. Patent Application Publication No. 2023/0030367, then entire contents of which is incorporated herein by reference. In at least one example embodiment, the draping system is attached relatively firmly to help avoid being caught by the consumable device.

For example purposes, the draping system according to example embodiments will be discussed with regard to a catheter-based procedure system, such as the catheter-based procedure system 10 shown in FIG. 1. However, example embodiments should not be limited to this example.

According to at least one example embodiment, the draping system includes three separate (individual) drapes that cover the catheter-based procedure system from the left, the right, and the bottom when viewed from the patient or table side (viewing the handle side or the longitudinal wall of the drive body) of the catheter-based procedure system. With regard to the catheter-based procedure system 10 shown in FIG. 1, for example, the left (distal) drape and the right (proximal) drape are tube shaped to slide over the proximal and distal ends of the drive body 100, whereas the bottom (arm) drape is a flatter drape that is wrapped upward around the positioning system 22. Perforated tape may be used to maintain the tube shape of the distal drape and the proximal drape temporarily when deploying the respective drapes. After the drive body 100 is draped with a respective one of the distal and proximal drape, the perforated tape is torn off to expose the channel on the bottom wall 114 of the drive body 100. Each of the distal and proximal drapes includes a front plate and a bottom plate with fasteners (e.g., magnets and/or mechanical snaps) that may keep the drapes away from the movable device modules 32a-32d. The arm drape is clipped onto the rotational joint 22a below the drive body 100 and then wrapped upward around the remaining portions of the positioning system 22. The arm drape is held in position around the positioning system with various hook and loop fasteners.

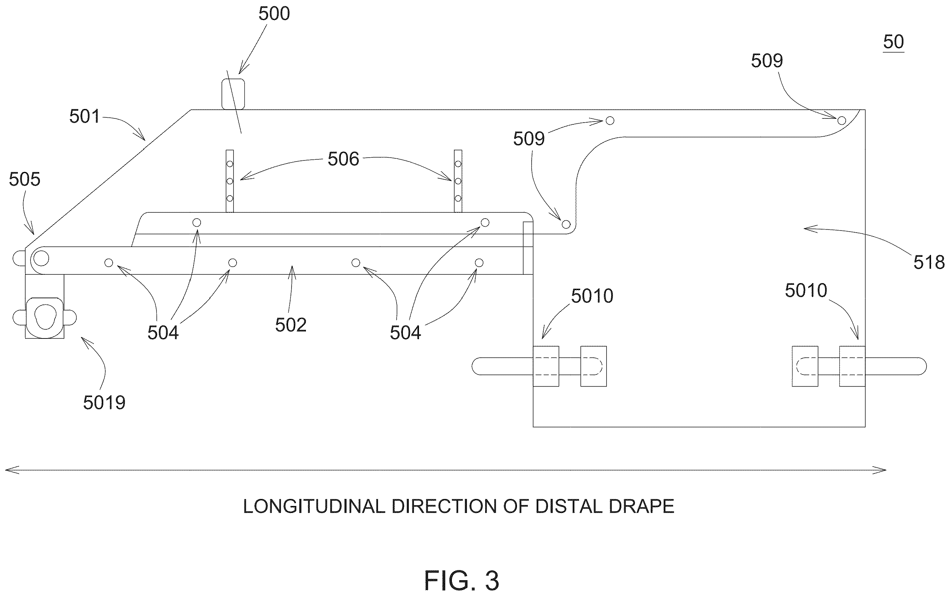

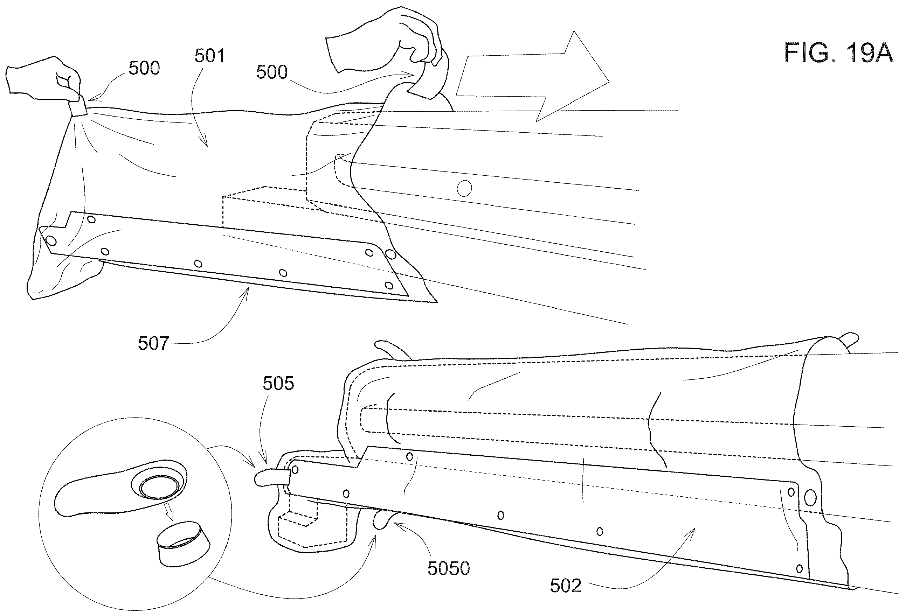

An example embodiment of the draping system will now be discussed in more detail below with regard to FIGS. 3-18 and 20. A method of draping the catheter-based procedure system 10, according to an example embodiment, will then be described with regard to FIGS. 19A-19J.

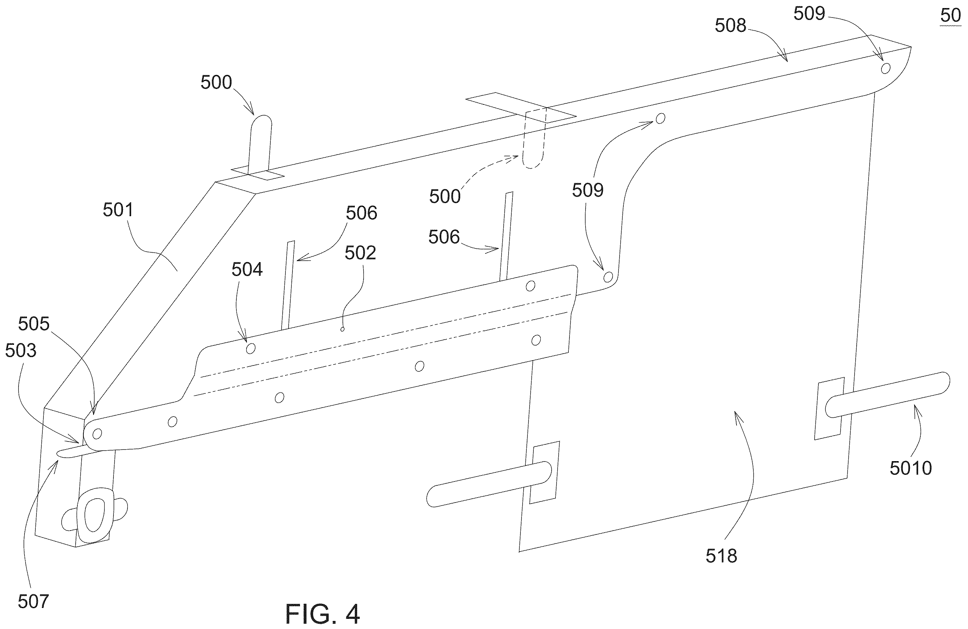

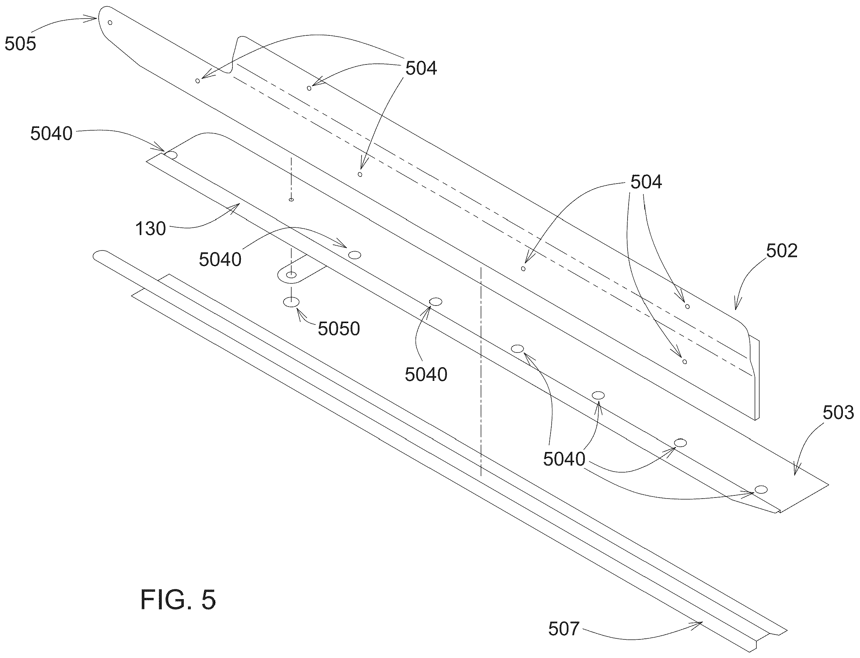

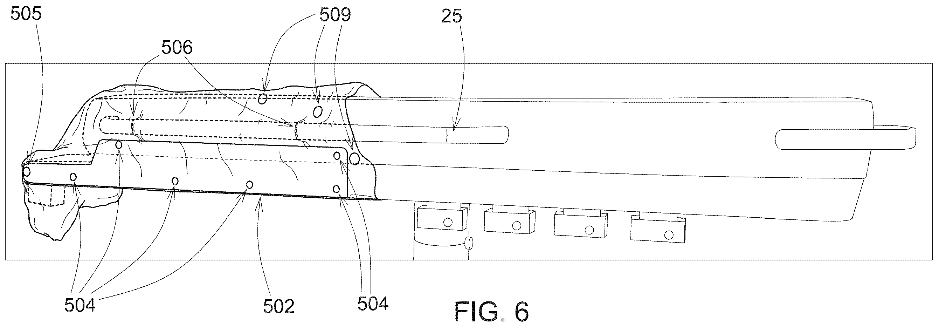

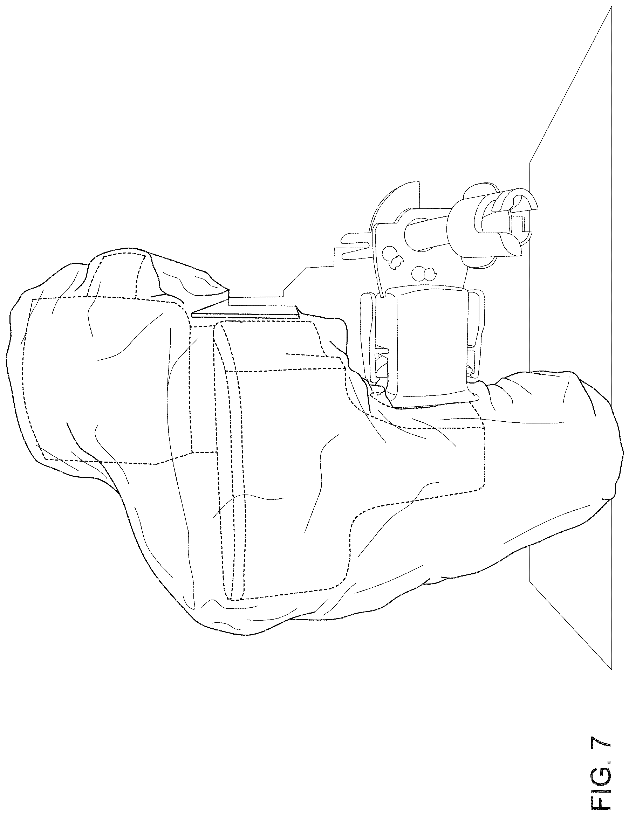

FIG. 3 is an inside plan view of a distal drape 50 (also referred to as a left or first tubular drape) according to example embodiments. FIG. 4 is a front perspective view of the distal drape 50 shown in FIG. 3. FIG. 5 is an exploded view of the distal rigid front plate, the distal rigid bottom plate and the distal perforated tape of the distal drape shown in FIGS. 3 and 4. FIG. 6 is a rear perspective view of a portion of the catheter-based procedure system 10 having the distal drape 50 deployed thereon. FIG. 7 is a front view of the catheter-based procedure system having the distal drape 50 deployed thereon.

Referring to FIGS. 3-7, the distal drape 50 includes a flexible distal drape sheet 501 (also referred to as a flexible distal drape portion or drape body) configured to cover the distal wall 106 and a distal portion of the top wall 102, the first longitudinal wall 108 and the second longitudinal wall 110 of the drive body 100. The distal drape sheet 501 may be formed from a polyethylene material having a thickness of 0.002 inches (0.00508 cm). However, example embodiments should not be limited to this example.

The distal drape 50 further includes a rigid front plate 502 secured to a first bottom edge portion the distal drape sheet 501. The rigid front plate 502 extends in a longitudinal direction to cover a lower distal portion of the first longitudinal wall 108 when deployed on the drive body 100. As shown in FIGS. 6 and 7, in one example, the rigid front plate 502 may extend from the distal end of the first longitudinal wall 108 half or about half the length of the drive body 100. When deployed, the rigid front plate 502 may extend up from the bottom edge of the longitudinal wall 108. A top edge of the rigid front plate 502 may sit below the bottom edge of the handle 25. In one example, the rigid front plate 502 may be formed from a Polyethylene Terephthalate Glycol (PETG) material having a thickness of 0.03 inches (0.0762 cm). However, example embodiments should not be limited to this example.

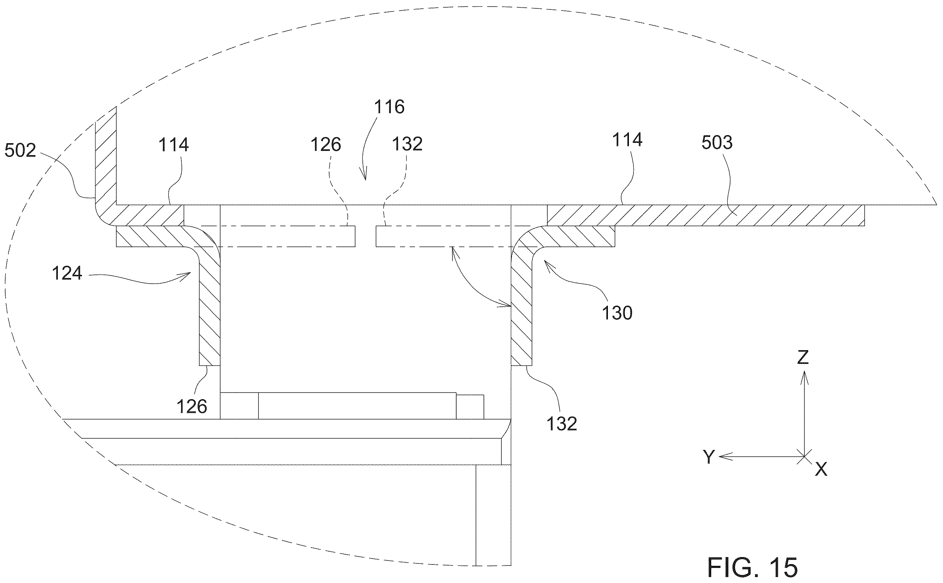

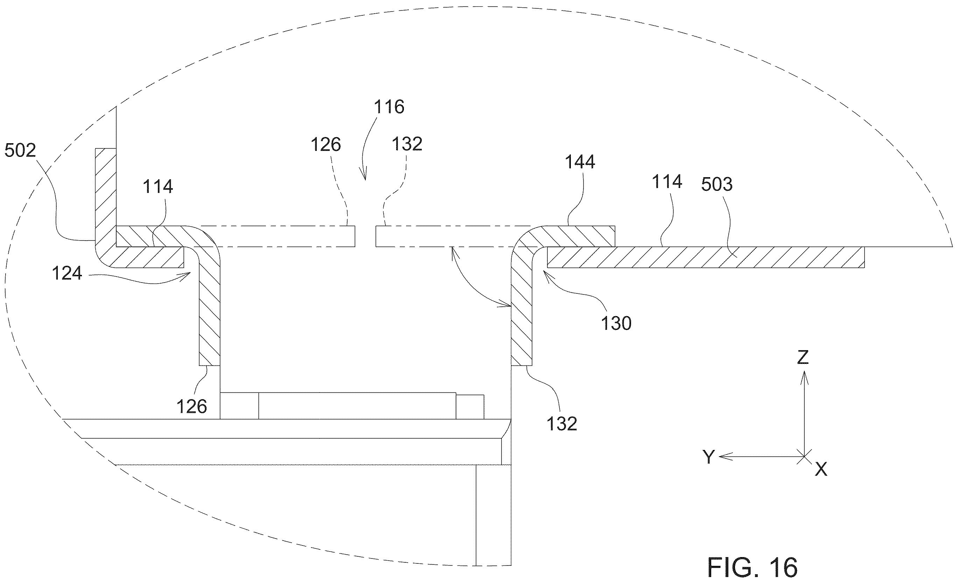

As described in more detail later with regard to FIGS. 15 and 16, the rigid front plate 502 may be L-shaped when viewed in the longitudinal direction so as to cover a portion (e.g., relatively small portion or edge) of the bottom wall 114. A first resilient member 124 is fixed along the edge of the L-shaped portion of the rigid front plate 502. The first resilient member 124 has a first free edge 126 (FIGS. 15 and 16) proximate a longitudinal axis of the drive body 100 along which the device modules 32a-32d move. The first resilient member 124 extends along the entire edge of the rigid front plate 502.

The rigid front plate 502 includes a snap cap 505 and magnets 504 to temporarily secure or fix the rigid front plate 502 to the first longitudinal wall 108 of the drive body 100 when deployed. The drive body 100 may have a corresponding snap cap and corresponding magnets or magnetic material at positions where the snap cap 505 and magnets 504 are to attach to the drive body 100. In the example embodiment shown in FIGS. 3-7, the rigid front plate 502 includes a snap cap 505 at a distal end and six magnets 504 arranged along the length and height of the rigid front plate 502.

The distal drape 50 also includes a distal rigid bottom plate 503 secured to a second bottom edge portion of the distal drape sheet 501. The second bottom edge portion of the distal drape sheet 501 may be opposite the first bottom edge portion at the bottom of the distal drape sheet 501. The rigid bottom plate 503 may extend in the longitudinal direction from the distal end of the bottom wall 114 half or about half the length of the drive body 100. In one example, the rigid bottom plate 503 may be the same or substantially the same length as the rigid front plate 502 and/or may be formed from a PETG material having a thickness of 0.03 inches (0.0762 cm). However, example embodiments should not be limited to this example.

A second resilient member 130 (FIGS. 15 and 16) is fixed along the edge of the rigid bottom plate 503. The second resilient member 130 has a first free edge 132 (FIGS. 15 and 16) proximate the longitudinal axis along which the device modules 32a-32d move during operation. Second resilient member 130 extends along the entire edge of the rigid bottom plate 503.

The rigid bottom plate 503 includes magnets 5040 spaced apart along the length of the plate to temporarily secure or fix the rigid bottom plate 503 to the bottom wall 114 of the drive body 100. The bottom wall 114 of the drive body 100 may have corresponding magnets or magnetic material at positions where the magnets 5040 are to attach to the drive body 100. In the example embodiment shown in FIGS. 3-7, the rigid bottom plate 503 includes seven magnets 5040 arranged along the length of the rigid bottom plate 503.

The rigid bottom plate 503 also includes a snap cap 5050 to further secure or fix the rigid bottom plate 503 to the bottom wall 114 of the drive body 100. The bottom wall 114 of the drive body 100 may have a corresponding snap socket at a position where the snap cap 5050 is to attach to the bottom wall 114. In the example embodiment shown in FIGS. 3-7, the snap cap 5050 is arranged towards the distal end of the rigid bottom plate 503 such that when the distal drape 50 is deployed, the snap cap 5050 is positioned toward the distal end of the drive body 100.

Still referring to FIGS. 3-7, an edge of the rigid front plate 502 is temporarily secured to an edge of the rigid bottom plate 503 via a perforated tape 507 to maintain a tube shape for deploying the drape on the robotic drive 24. In one example, the perforated tape 507 may be Low-Density Polyethylene (LDPE) with two laminated adhesive lines on the tape and without adhesive in the middle of the tape. In this example, the adhesive lines adhere to the rigid front plate 502 and the rigid bottom plate 503, while not being adhered or attached to the respective resilient members 124 and 130.

The first and second resilient members 124 and 130 will be discussed in more detail later with regard to FIGS. 15, 16 and 18.

A plurality of (e.g., two) twisted ties 506 may be fixed to the front portion of the distal drape sheet 501 above the rigid front plate 502. The twisted ties 506 may be composed of a thin bendable metal and function as bendable tethers to at least partially wrap the distal drape sheet 501 around the handle 25.

As mentioned above, the perforated tape 507 may connect the rigid front plate 502 and the rigid bottom plate 503 along the length of plates to form a tube-shape. After the drape is deployed, this perforated tape is torn off exposing a portion of the channel on the bottom wall 114 of the drive body 100.

Still referring to FIGS. 3-7, the distal drape sheet 501 further includes an overlapping portion 508 and a rear positioning system covering portion 518. The overlapping portion 508 overlaps with the proximal drape 52 after the proximal drape 52 is deployed. The overlapping drape portion 508 includes hook or loop portions 509 of hook and loop fasteners that engage with corresponding hook or loop portions on the proximal drape 52 to secure the overlapping drape portion 508 to the proximal drape 52 when deployed. The rear positioning system covering portion 518 covers the top of the positioning system 22, for example, after deploying the arm drape 54 to cover the underside of the positioning system 22. The rear arm covering portion 518 includes handle straps 5010 with hook and loop fasteners to wrap and secure the rear positioning system covering portion 518 around the arm drape after the arm drape 54 is deployed.

The distal drape 50 may include one or more tabs 500 to facilitate draping of the robotic drive 24 by a user as described later. The user may utilize the tabs to hold and maneuver the distal drape 50 during draping.

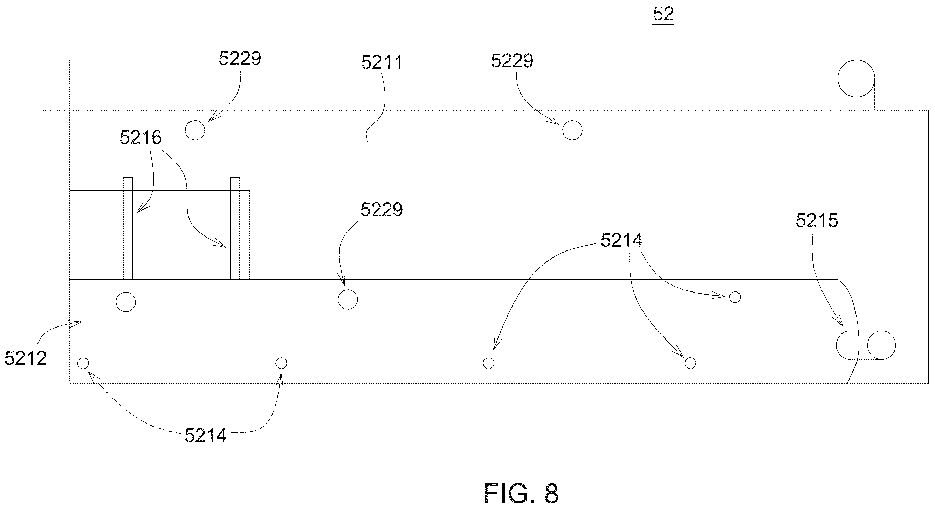

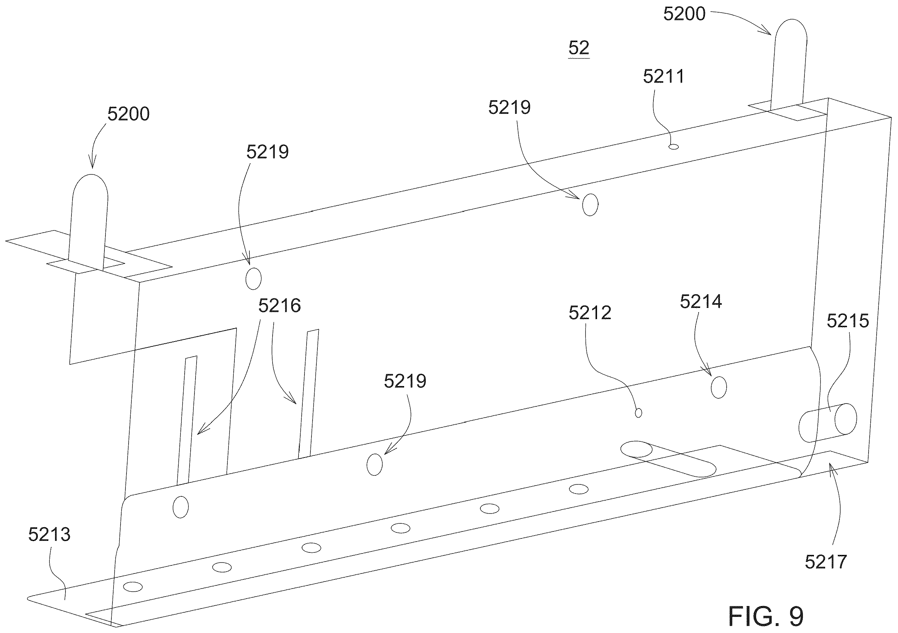

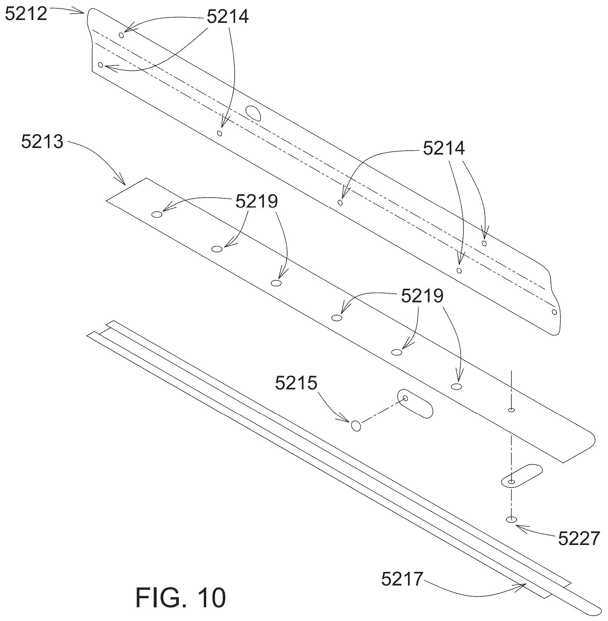

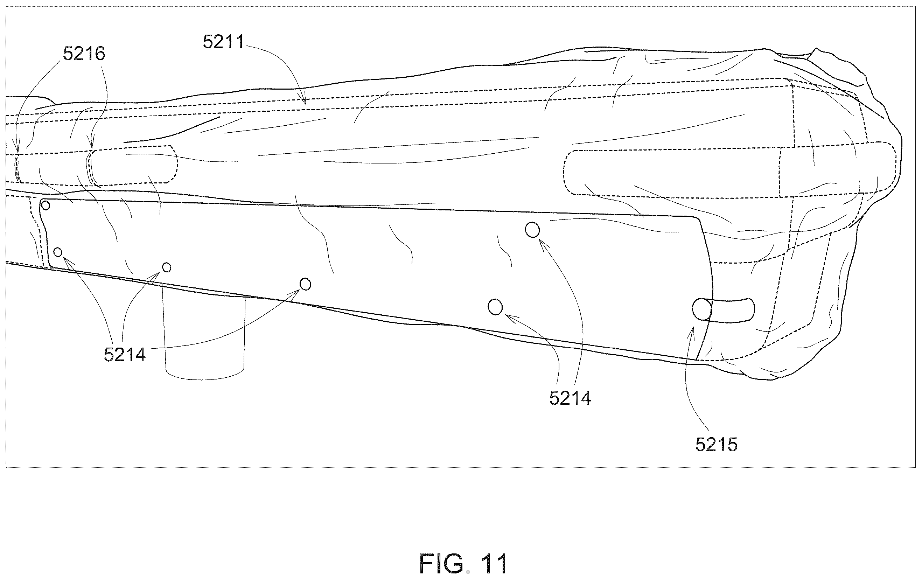

FIG. 8 is an inside plan view of a proximal drape 52 (also referred to as a right or second tubular drape) according to example embodiments. FIG. 9 is a front perspective view of the proximal drape 52 shown in FIG. 8. FIG. 10 is an exploded view of the proximal rigid front plate, the proximal rigid bottom plate and the proximal perforated tape of the proximal drape 52 shown in FIGS. 8 and 9. FIG. 11 is a rear perspective view of a portion of the catheter-based procedure system10 having the proximal drape 52 deployed thereon.

Referring to FIGS. 8-11, the proximal drape 52 includes a flexible proximal drape sheet 5211 (also referred to as a flexible proximal drape portion or drape body) configured to cover proximal wall 104 and a proximal portion of the top wall 102, first longitudinal wall 108 and second longitudinal wall 110 of drive body 100. As noted above, the proximal drape sheet 5211 may overlap with the distal drape sheet 501 along the top wall 102 of the drive body 100 when deployed. The proximal drape sheet 5211 may be formed from a polyethylene material having a thickness of 0.002 inches (0.00508 cm). However, example embodiments should not be limited to this example.

The proximal drape 52 includes a proximal rigid front plate 5212 secured to a first bottom edge portion of the proximal drape sheet 5211. The rigid front plate 5212 extends in a longitudinal direction to cover a proximal lower portion of the first longitudinal wall 108 when draped on the drive body 100. As shown in FIG. 11, the rigid front plate 5212 may extend from the proximal end of the first longitudinal wall 108 half or about half the length of the drive body 100. When deployed, the rigid front plate 5212 may extend up from the bottom of the longitudinal wall 108. A top edge of the rigid front plate 5212 may sit below the bottom edge of the handle 25. In one example, the rigid front plate 5212 may be formed from a PETG material having a thickness of 0.03 inches (0.0762 cm). However, example embodiments should not be limited to this example. In one example, the rigid front plates 502 and 5212 may have the same vertical height.

As described later with regard to FIGS. 15 and 16, the rigid front plate 5212 may be L-shaped when viewed in the longitudinal direction so as to cover a portion (e.g., relatively small portion or edge) of the proximal portion of the bottom wall 114. A first resilient member 124 is also fixed along the edge of the L-shaped portion of the rigid front plate 5212. The first resilient member 124 again has a first free edge 126 proximate a longitudinal axis of the drive body 100 along which the device modules 32a-32d move. The first resilient member 124 extends along the entire edge of the rigid front plate 5212.

The rigid front plate 502 includes a snap cap 5215 and magnets 5214 to temporarily secure or fix the rigid front plate 5212 to the proximal portion of the first longitudinal wall 108 of the drive body 100. The drive body 100 may have a corresponding snap cap and corresponding magnets or magnetic material at positions where the snap cap 5215 and magnets 5214 are to attach to the drive body 100. In the example embodiment shown in FIGS. 8-10, the rigid front plate 5212 includes a snap cap 5215 at a proximal end and six magnets 5214 arranged along the length and height of the rigid front plate 5212.

The proximal drape 52 also includes a proximal rigid bottom plate 5213 secured to a second bottom edge portion of the proximal drape sheet 5211. The second bottom edge portion of the proximal drape sheet 5211 may be opposite the first bottom edge portion at the bottom of the proximal drape sheet 5211. The rigid bottom plate 5213 may extend in the longitudinal direction from the proximal end of the bottom wall 114 half or about half of the length of the drive body 100 to meet the rigid bottom plate 503 when deployed. In one example, the rigid bottom plate 5213 may be the same or substantially the same length as the rigid front plate 5212 and/or may be formed from a polycarbonate material having a thickness of 0.03 inches (0.0762 cm). However, example embodiments should not be limited to this example.

A second resilient member 130 is also fixed along the edge of the rigid bottom plate 5213. The second resilient member 130 has the first free edge 132 proximate the longitudinal axis along which the device modules 32a-32d move during operation. Second resilient member 130 extends along the entire edge of the rigid bottom plate 5213. Although described with regard to the proximal drape 52 including resilient members for example purposes, example embodiments should not be limited to this example. Rather, one or more resilient members may be omitted from the proximal drape 52.

The rigid bottom plate 5213 includes magnets 5219 spaced apart along the length of the plate to temporarily secure or fix the rigid bottom plate 5213 to the bottom wall 114 of the drive body 100. The bottom wall 114 of the drive body 100 may have corresponding magnets or magnetic material at positions where the magnets 5219 are to attach to the drive body 100. In the example embodiment shown in FIGS. 8-10, the rigid bottom plate 5213 includes six magnets 5219 arranged along the length of the rigid bottom plate 5213. The rigid bottom plate 5213 may also include a snap cap 5227 to engage a corresponding snap socket on the bottom wall 114 to provide additional security when deploying the proximal drape 52 on the drive body 100.

As with the proximal drape 52, an edge of the rigid front plate 5212 is temporarily secured to an edge of the rigid bottom plate 5213 via a perforated tape 5217 to maintain the tubular shape of the proximal drape 52.

A plurality of (e.g., two) twisted ties 5216 may be fixed to the front portion of the proximal drape sheet 5211 above the rigid front plate 5212. The twisted ties 5216 may be the same or substantially the same as the twisted ties 506 discussed above and may function as bendable tethers to at least partially wrap the proximal drape sheet 5211 around a proximal portion of the handle 25.

As with the distal drape 50, after the proximal drape 52 is deployed, the perforated tape 5217 is torn off exposing a portion of the channel on the bottom wall 114 of the drive body 100. The perforated tape 5217 may be the same or substantially the same as the perforated tape 507 discussed above with regard to the distal drape 50.

The proximal drape 52 may further include hook or loop portions 5229 corresponding to the hook or loop portions 509 on the distal drape 50. The hook or loop portions 5229 engage with the corresponding hook or loop portions 509 on the distal drape 50 to secure the overlapping drape portion 508 to the proximal drape 52 on deployment.

The proximal drape 52 may include one or more (e.g., two) tabs 5200 to facilitate draping of the robotic drive 24 by a user. The user may utilize the tabs 5200 to hold and maneuver the proximal drape 52 during draping.

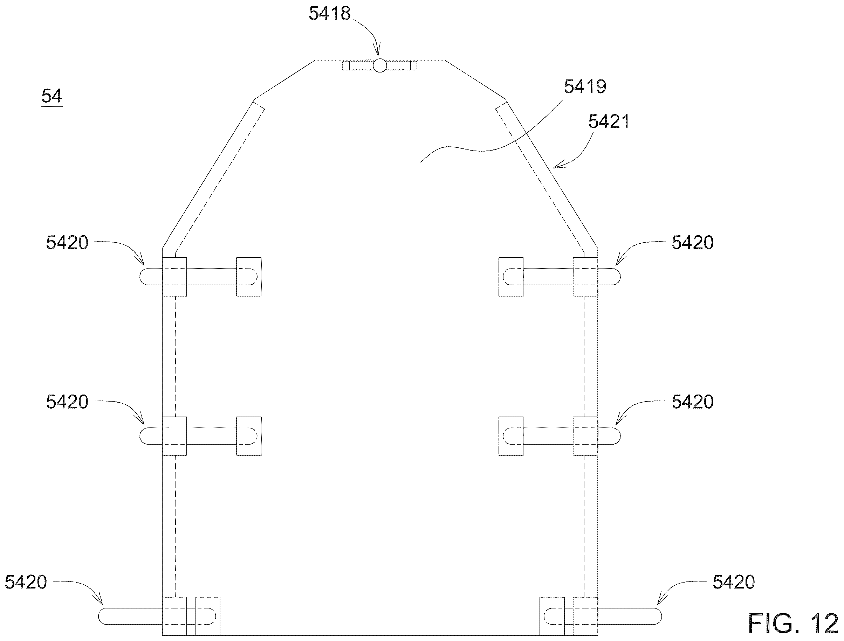

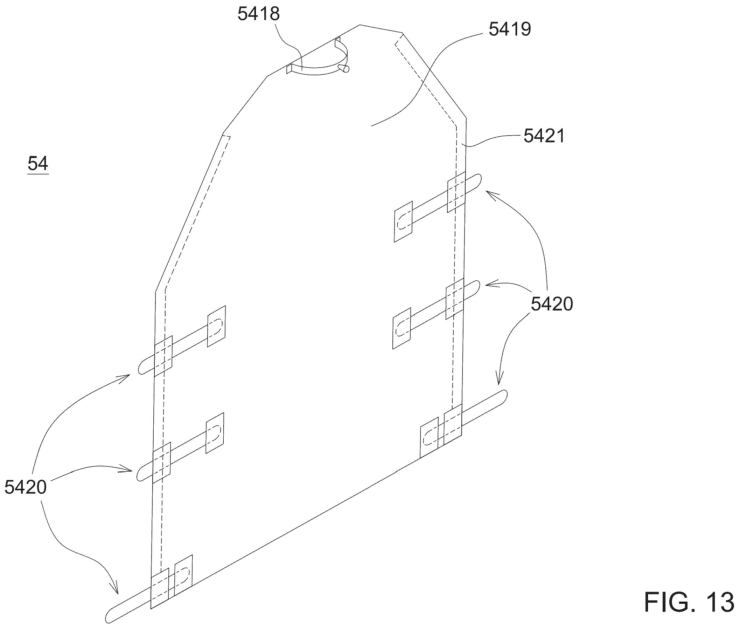

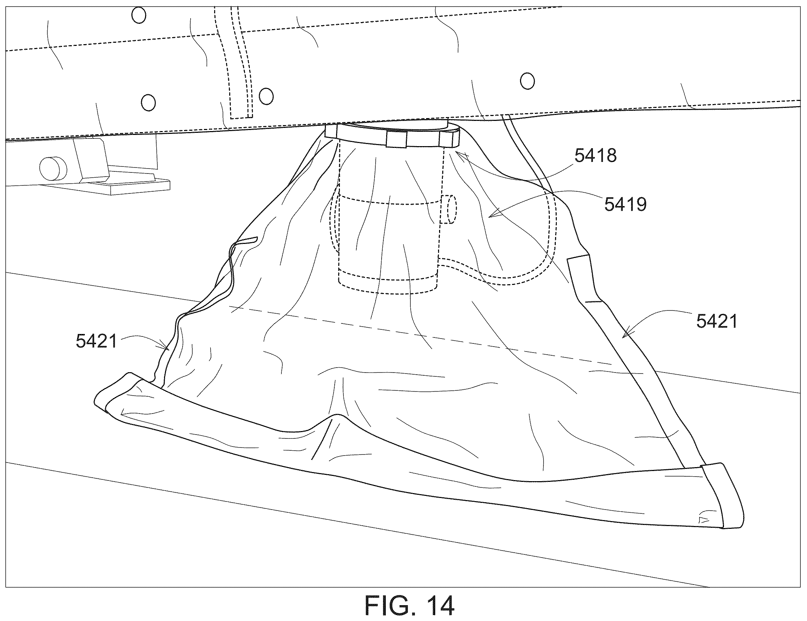

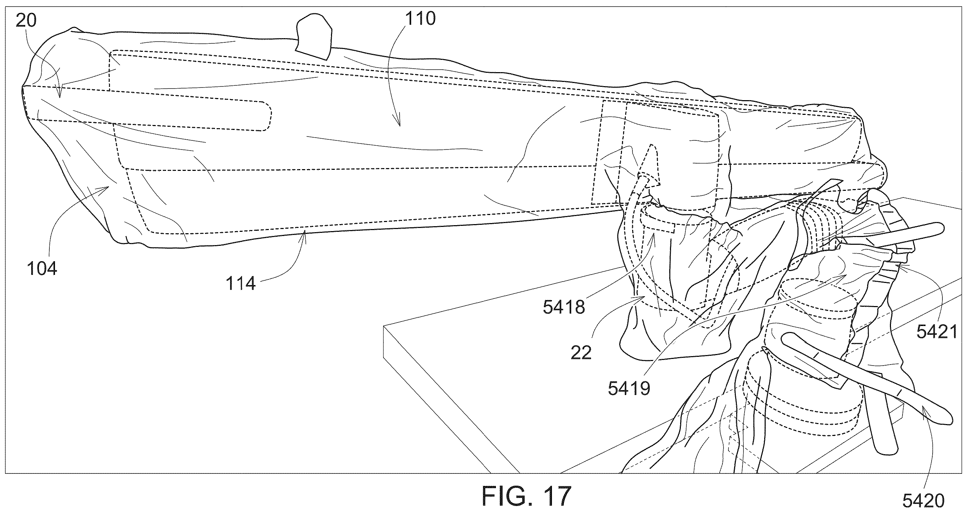

FIG. 12 is a plan view of an arm drape 54 (also referred to as a non-tubular drape or non-tubular third drape) according to example embodiments. FIG. 13 is a perspective view of the arm drape 54 shown in FIG. 12. FIG. 14 is a view of the arm drape 54 partially attached to the positioning system 22 of the catheter-based procedure system 10. FIG. 17 is a perspective view of an example embodiment of the draping system deployed on the catheter-based procedure system 10.

Referring to FIGS. 12-14 and 17, the arm drape 54 includes a flexible arm drape sheet 5419 (also referred to as flexible arm drape portion or drape body) to wrap around the joints and arms of the positioning system 22 to provide a sterile barrier between the patient 12 and the positioning system 22 during a medical procedure. The arm drape sheet 5419 may be formed from a polyethylene material having a thickness of 0.002 inches (0.00508 cm). However, example embodiments should not be limited to this example.

A lineal tape 5421 having a continuous hook and loop fastener system may be fixed along opposite edges of the arm drape sheet 5419. In this regard, a hook portion may be fixed to one edge (e.g., the left edge) of the arm drape 54 and the loop portion may be fixed to the other edge (e.g., the right edge) of the arm drape 54 to provide a seamless seal when wrapped around the positioning system 22. A plurality of hand straps 5420 may be fixed along each of the edges of the arm drape sheet 5419. In the example embodiment shown in FIGS. 12-13, three hand straps 5420 are fixed to each edge of the arm drape sheet 5219. The hand straps 5420 may be used to hold and/or secure the arm drape 54 around the positioning system 22 during draping to facilitate creating the seamless seal using the hook and loop fastening system along the edges of the arm drape 54.

A c-shaped clip 5418 may be fixed to an upper portion or end of the arm drape sheet 5419. The c-shaped clip 5418 may be used to couple the arm drape 54 to the rotational joint 22a of the positioning system 22 under the robotic drive 24 as shown, for example, in FIGS. 14 and 17. The c-shaped clip may be the same or substantially the same as that described in U.S. Patent No. 11,903,669, the entire contents of which are incorporated herein by reference.

The rigid plates, the resilient members and the channel exposed by removal of the perforated tape on the bottom wall 114 of the drive body 100 will now be described with regard to FIGS. 15, 16 and 18. The following discussion is made with regard to the distal rigid front plate 502 and the distal rigid bottom plate 503. It should be understood, however, that the same discussion applies to the proximal rigid front plate 5212 and the proximal rigid bottom plate 5213.

FIG. 15 is a cross-sectional view of an example embodiment of the rigid front plate 502 and the rigid bottom plate 503 deployed on the drive body 100. The cross-section shown in FIG. 15 is taken at a point orthogonal to the longitudinal direction of the distal drape 50 at any point along the rigid front plate 502 and the rigid bottom plate 503, and is a close up view of the drape on the catheter-based procedure system 10 shown in FIG. 2 taken generally along line 7-7. For example purposes, FIG. 15 is discussed with regard to drive module 32d.

Referring to FIG. 15, a portion of first free edge 126 adjacent the drive module 32d is resiliently biased away from and returned to the longitudinal axis as the drive module 32d moves along the longitudinal axis. Only a portion of first free edge 126 of first resilient member 124 that is closely adjacent drive module 32d is biased away from the longitudinal axis. Stated another way, the portion of first free edge 126 of first resilient member 124 that is distal to drive module 32d is not biased away from the longitudinal axis. By way of example, and referring to FIG. 18, a region of first free edge 126 proximate point A that is closely adjacent drive module 32d is biased away from the longitudinal axis of the drive body 100. Once drive module 32d moves along the longitudinal axis such that region of first free edge 126 proximate point A is no longer closely adjacent drive module 32d the region of first free edge 126 proximate point A will resiliently return to the longitudinal axis.

As with the first free edge 126 of the first resilient member 124, a portion of first free edge 132 adjacent the drive module 32d is resiliently biased away from and returned to the longitudinal axis as drive module 32d moves along the longitudinal axis. Only a portion of first free edge 132 of second resilient member 130 that is closely adjacent drive module 32d is biased away from the longitudinal axis. Stated another way, the portion of first free edge 132 of second resilient member 130 that is distal drive module 32d is not biased away from the longitudinal axis. Again, by way of example and referring to FIG. 18, a region of first free edge 132 proximate point A that is closely adjacent drive module 32d is biased away from the longitudinal axis. Once drive module 32d moves along the longitudinal axis such that region of first free edge 132 proximate point A is no longer closely adjacent drive module 32d the region of first free edge 132 proximate point A will resiliently return to the longitudinal axis.

First resilient member 124 and second resilient member 130 may be formed of EPDM (Ethylene Propylene Diene Monomer) rubber having a thickness of 1/32 inch (0.0794 cm). First rigid member 146 has a rigidity and a resiliency greater than the flexible drape portions.

FIG. 16 is a cross-sectional view of another example embodiment of the distal rigid front plate 502 and the distal rigid bottom plate 503 deployed on the robotic drive 24. As with the example embodiment shown in FIG. 15, the cross-section shown in FIG. 16 is taken at a point orthogonal to the longitudinal direction of the distal drape 50 at any point along the rigid front plate 502 and the rigid bottom plate 503. The discussion of the example embodiment shown in FIG. 16 may also apply to the proximal rigid front plate 5212 and the proximal rigid bottom plate 5213.

The example embodiment shown in FIG. 16 is similar to the example embodiment shown in FIG. 15, except that the first resilient member 124 is arranged on the opposite side of the rigid front plate 502 such that the first resilient member 124 is between the edge of the rigid front plate 502 and the bottom wall 114 of the drive body 100 when deployed. Similarly, the second resilient member 130 is arranged on the opposite side of the rigid bottom plate 503, relative to the example embodiment shown in FIG. 15, such that the second resilient member 130 is between the edge of the rigid bottom plate 503 and the bottom wall 114 of the drive body 100when deployed. Other aspects of the example embodiment shown in FIG. 16 are the same or substantially the same as the example embodiment shown in FIG. 15. As such, a detailed discussion is omitted.

A method for deploying the draping system according to one or more example embodiments will now be described.

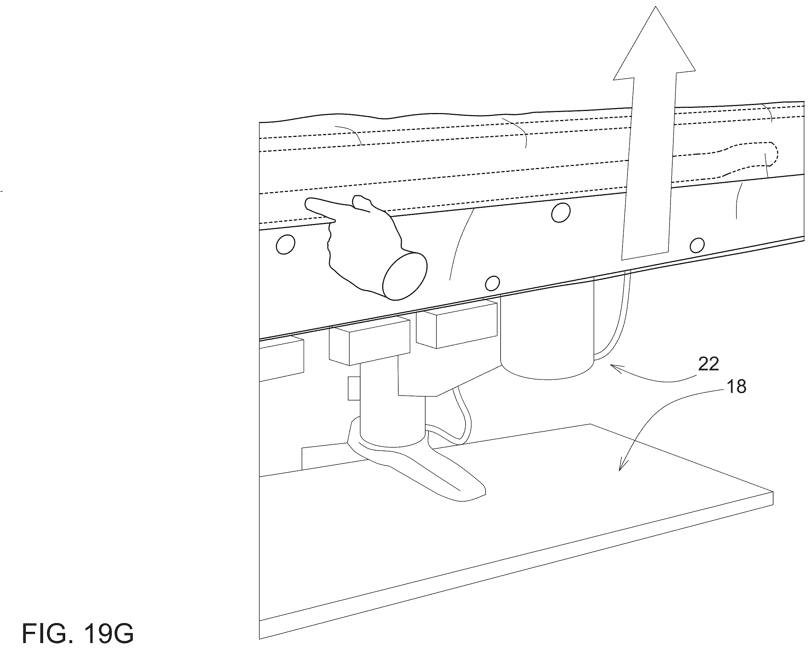

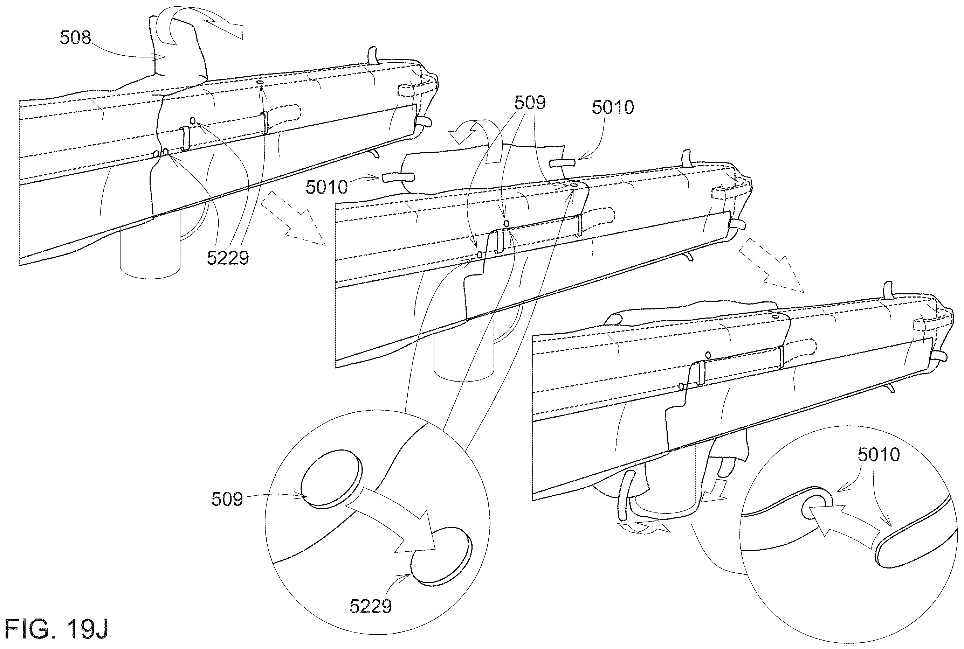

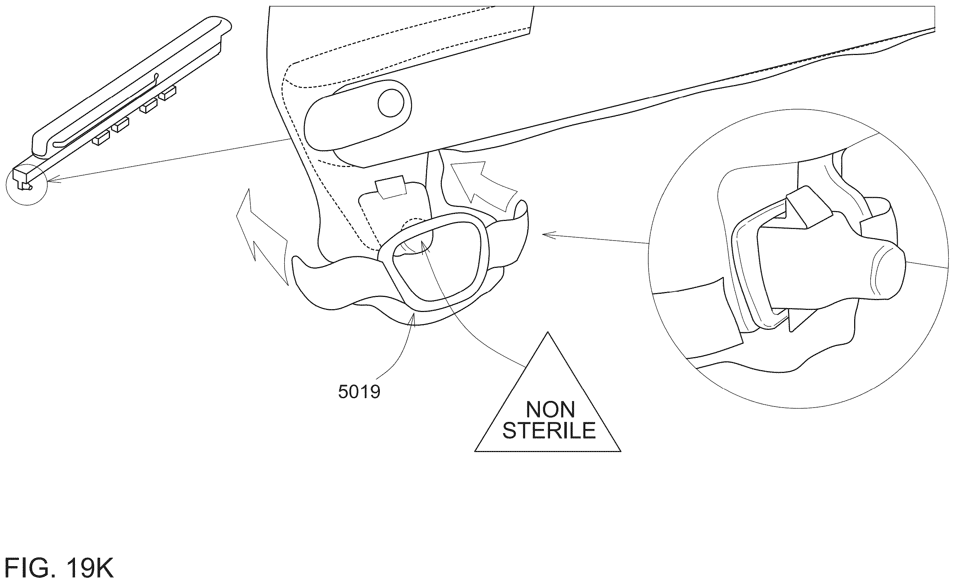

FIGS. 19A-19K illustrate a method for deploying, on the catheter-based procedure system 10, the draping system described herein with regard to one or more example embodiments. Prior to deploying the drape in the manner discussed with regard to FIGS. 19A-19K, the drive modules 32a-32d are moved to the proximal end of the robotic drive 24. As discussed in more detail below, FIGS. 19A-19F, 19J and 19K generally illustrate an example embodiment of a method for deploying the distal drape 50 and the proximal drape 52. FIGS. 19G-19I generally illustrate an example embodiment of a method for deploying the arm drape 54.

Referring to FIG. 19A, a user, operator or technician slides the distal drape 50 onto the distal portion of the robotic drive 24. In one example, the user holds the distal drape 50 at the top portion (e.g., via tabs 500 fixed to the top of the drape) and slides the distal drape 50 onto the drive body 100 from the distal end (distal longitudinal end) of the drive body 100. As the user slides the distal drape 50 onto the drive body 100, the magnets 504, 5040 on the rigid front plate 502 and the rigid bottom plate 503 snap the plates into alignment on the robotic drive 24 to initially secure the distal drape 50 on the robotic drive 24. The user then further secures the distal drape 50 by engaging the snap caps 505 and 5050 on the rigid front plate 502 and the rigid bottom plate 503, respectively.

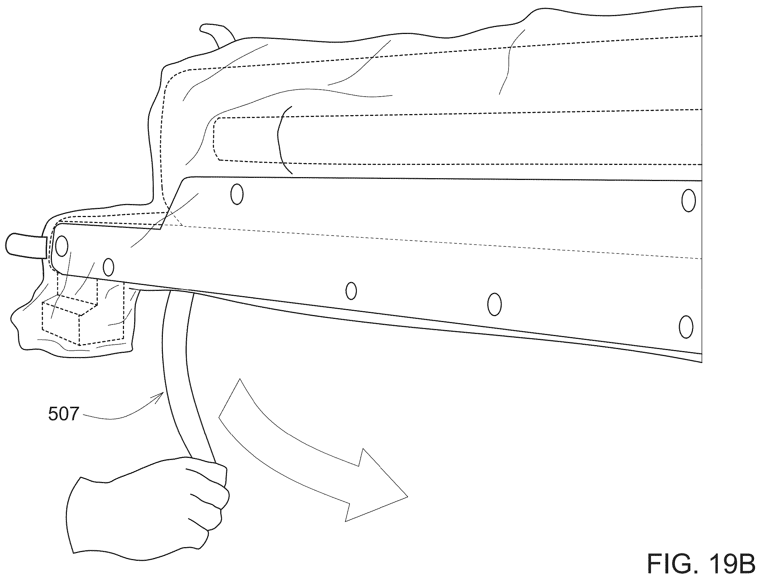

As shown in FIG. 19B, after the distal drape 50 is arranged on the drive body 100, the user removes the perforated tape 507 to expose the channel between the resilient members 124 and 130 on the bottom wall 114. In another example embodiment, the user need not remove the perforated tape 507 to expose the channel between the resilient members 124 and 130. Rather, the user may move at least drive module 32a through the channel to split the perforated tape 507 apart thereby creating the channel.

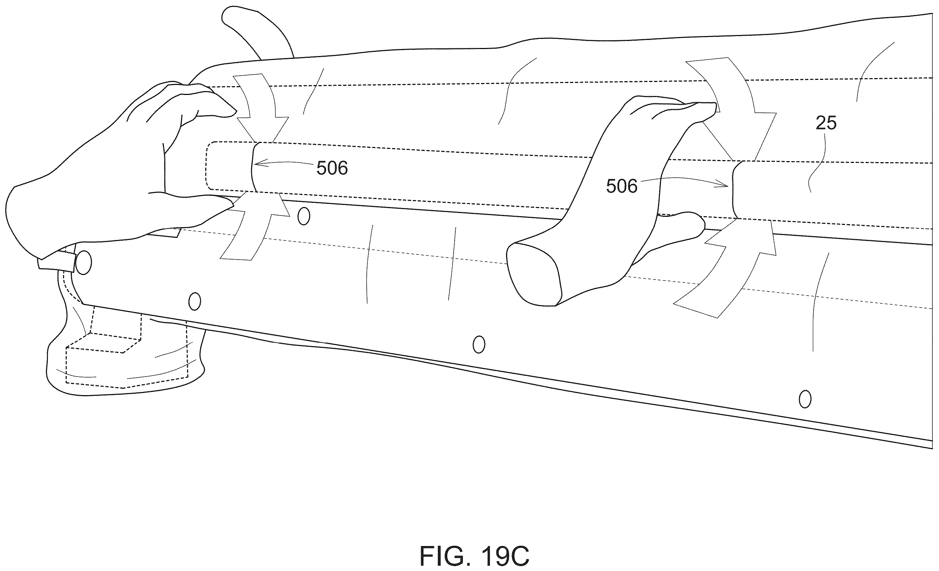

Once the perforated tape 507 is removed (or split apart), as shown in FIG. 19C the user squeezes or tightens the twist ties 506 around the handle 25 to pull the distal drape sheet 501 closer to the drive body 100. The overlapping drape portion 508 remains folded back over the remaining portion of the distal drape sheet 501 until the proximal drape 52 is deployed.

The user then moves the drive modules 32a-32d (or remaining ones of the drive modules 32b-32d) through the channel to the distal end of the robotic drive 24 before deploying the proximal drape 52.

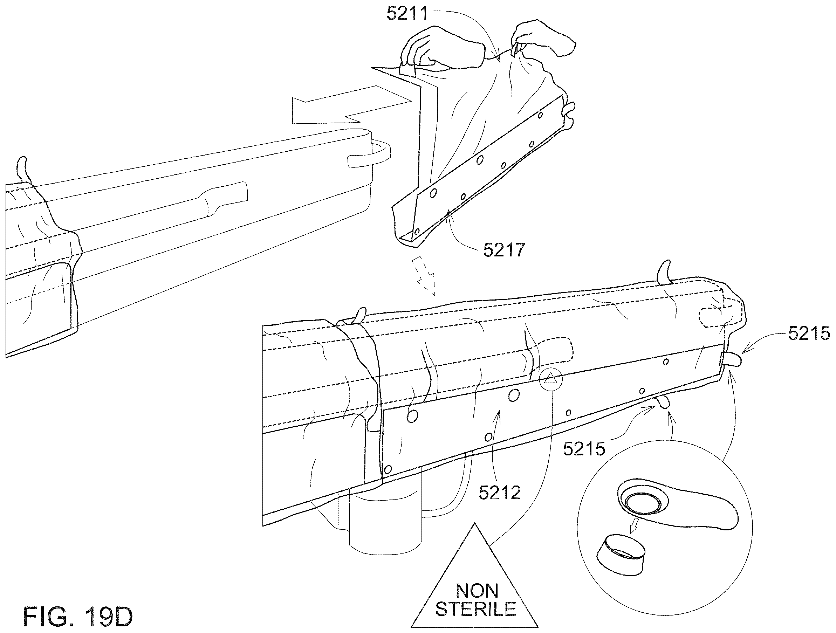

As shown in FIG. 19D, the user slides the proximal drape 52 onto the proximal portion of the drive body 100. In one example, the user holds the proximal drape 52 at the top portion (e.g., via tabs 5200) and slides the proximal drape 52 onto the drive body 100 from the proximal end (proximal longitudinal end). As the user slides the proximal drape 52 onto the drive body 100, the magnets 5214, 5219 on the rigid front plate 5212 and the rigid bottom plate 5213 snap the plates into alignment on the drive body 100 to initially secure the proximal drape 52 on the drive body 100. The user then further secures the proximal drape 52 by engaging the snap caps 5215 on the rigid front plate 5212 and the rigid bottom plate 5213, respectively.

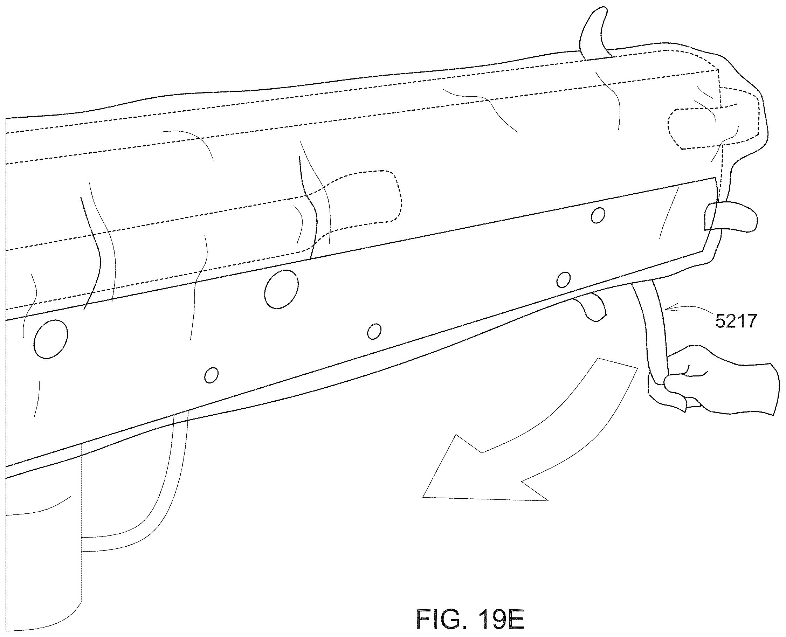

As shown in FIG. 19E, after the proximal drape 52 is arranged on the drive body 100, the user removes the perforated tape 5217 to expose the channel on the proximal portion of the bottom wall 114 between the front rigid plate 5212 and the rigid bottom plate 5213. In another example embodiment, the user need not remove the perforated tape 5217. Rather, the user may move at least drive module 32d through the channel to split the perforated tape 5217 apart thereby creating the channel.

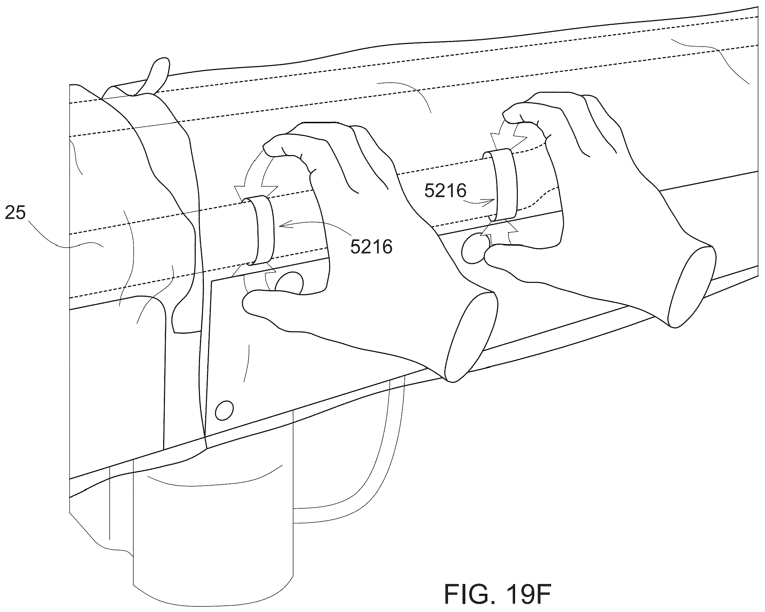

Once the perforated tape 5217 is removed (or split apart), as shown in FIG. 19F the user squeezes or tightens the twist ties 5216 around the proximal portion of the handle 25 to pull the proximal drape sheet 5211 closer to the drive body 100.

After tightening the twist ties 5216, as shown in FIG. 19G the user may raise (e.g., manually raise) the robotic drive 24 to increase vertical clearance between the robotic drive 24 and the patient table 18, and to increase accessibility to the positioning system 22 supporting the robotic drive 24.

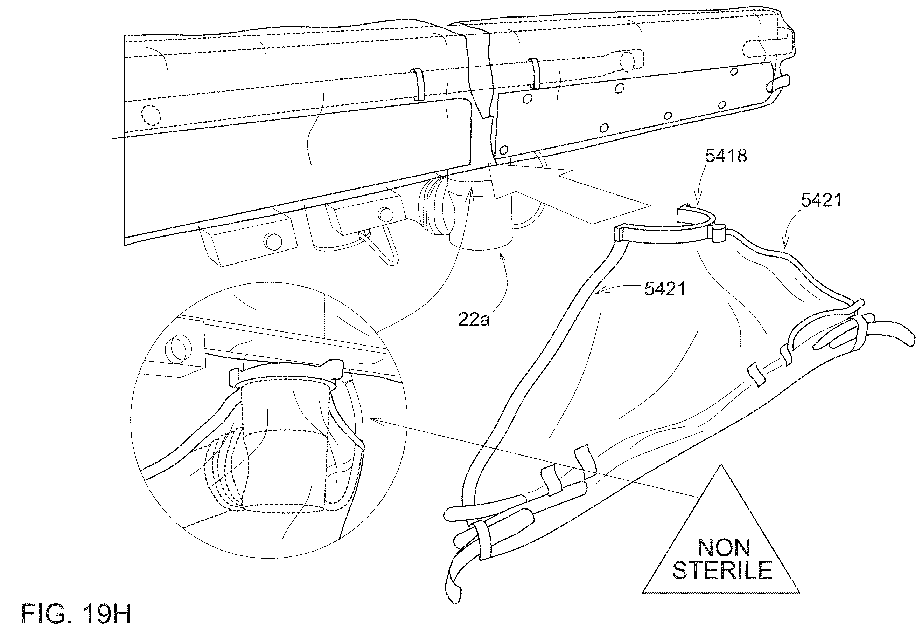

As shown in FIG. 19H, the user begins to deploy the arm drape 54 by attaching the c-shaped clip 5418 to the rotational joint 22a.

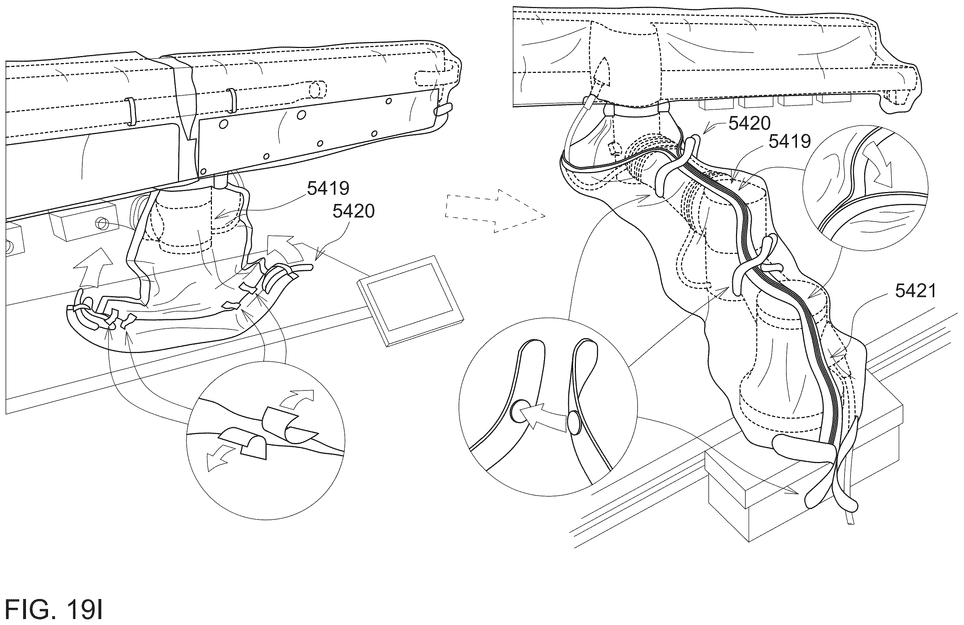

As shown in FIG. 19I, the user wraps the arm drape sheet 5419 around the support arm using the hand straps 5420. The user then secures the arm drape 54 around the support using the hook and loop fasteners on the hand straps 5420 and the hook and loop fastener system along the edges of the arm drape sheet 5419.

As shown in FIG. 19J, the user then folds the overlapping drape portion 508 over a portion of the proximal drape 52 and secures the overlapping drape portion 508 to the proximal drape 52 via the plurality of corresponding hook and loop fasteners on the distal drape 50 and the proximal drape 52. In the example embodiment shown in FIG. 19J, the overlapping drape portion 508 may be secured to the proximal drape 52 via at least three hook and loop fasteners. Still referring to FIG. 19J, the user may also cover the top of the positioning system 22 using the rear positioning system covering portion 518 of the distal drape 50.

As shown in FIG. 19K, the user then fits the sheath pull pad 5019 over a sheath anchor (also referred to as a device support connection). As shown, the sheath anchor has two tabs that prevent the sheath pull pad 5019 from bouncing back.

Although an example embodiment of a method for deploying the draping system is described with regard to the order of steps shown in FIGS. 19A-19K, example embodiments should not be limited to this example. Rather, the steps of the draping method may be performed in various different orders.

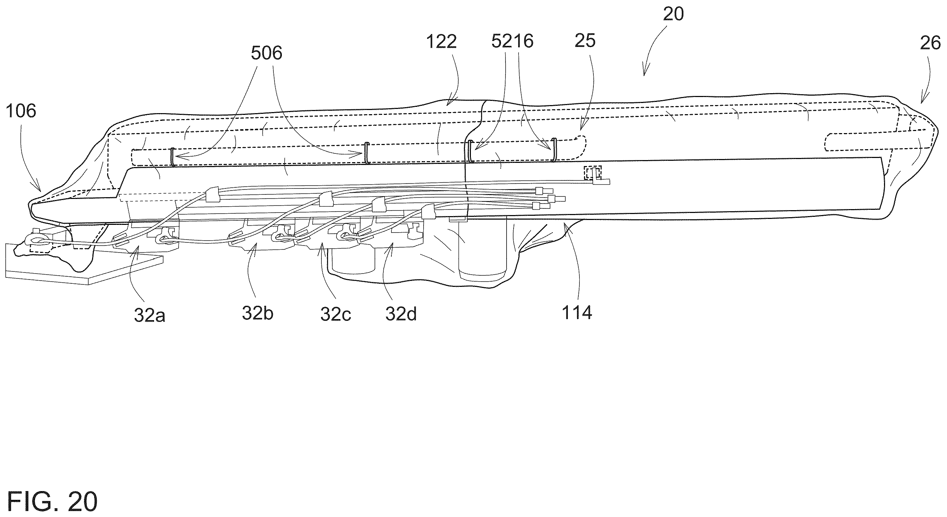

FIG. 20 is a view of the first longitudinal wall side of the catheter-based procedure system 10 draped with a draping system according to one or more example embodiments. Because aspects of the draping system shown in FIG. 20 have already been described, further discussion is omitted for the sake of brevity.

Draping systems according to one or more example embodiments described herein may be deployed within a relatively short amount of time (e.g., three minutes), which may reduce (e.g., significantly reduce) the draping time and training time required compared to a one-piece drape. The modular design of the draping systems, according to one or more example embodiments, may also simplify the manufacturing process, thereby reducing fabrication time and/or vendor costs. The design of the draping system according to one or more example embodiments may improve user experience through easier handling and/or deployment, while maintaining required functionality such as channel accessibility and secure the sheets without loose portions.

While magnets, snaps and hook and loop fasteners are indicated as some of the attaching features, other coupling methods known in the art are also contemplated. As a non-limiting example, for each of the attachment mechanisms, magnets, snaps, adhesive tape, hooks, hook and loop fasteners, and other mechanical, electro-mechanical and chemical coupling may be used in conjunction with or instead of the coupling methods described herein.

According to a first aspect, a draping system for a robotic drive having an elongated drive body and an articulating support arm supporting the elongated drive body is suggested, the draping system comprising: a first tubular drape configured to cover a first portion of the elongated drive body; a second tubular drape configured to cover a second portion of the elongated drive body, the second tubular drape being separate from the first tubular drape; and a third drape configured to cover the articulating support arm, the third drape being separate from the first tubular drape and the second tubular drape.

According to an embodiment, the first tubular drape is configured to slide onto the elongated drive body from a first end of the elongated drive body to cover the first portion of the elongated drive body.

According to a further embodiment, the second tubular drape is configured to slide onto the elongated drive body from a second end of the elongated drive body to cover the second portion of the elongated drive body, the second end being opposite the first end in a longitudinal direction of the elongated drive body.

According to a further embodiment, the first tubular drape includes a first perforated tape temporarily adhered along a length of the first tubular drape to maintain a tubular shape of the first tubular drape.

According to a further embodiment, the first perforated tape is configured to be removed after the first tubular drape is deployed on the elongated drive body.

According to a further embodiment, the robotic drive includes one or more movable drive modules configured to move along a length of the elongated drive body via a channel formed along a length of a bottom wall of the elongated drive body, and removal of the first perforated tape exposes the channel.

According to a further embodiment, the first tubular drape includes a first rigid plate and a second rigid plate extending along a length of the first tubular drape, and wherein the first perforated tape is temporarily adhered to the first rigid plate and the second rigid plate to maintain the tubular shape of the first tubular drape.

According to a further embodiment, the first tubular drape includes a flexible drape body, and wherein the first rigid plate and the second rigid plate are adhered to respective edges of the flexible drape body.

According to a further embodiment, the second tubular drape has a third rigid plate, a fourth rigid plate and a second perforated tape extending along a length of the second tubular drape, the second perforated tape being temporarily adhered to the third rigid plate and the fourth rigid plate to maintain a tubular shape of the second tubular drape.

According to a further embodiment, the second tubular drape includes a flexible drape body, and wherein the third rigid plate and the fourth rigid plate are adhered to respective edges of the flexible drape body.

According to a further embodiment, the third drape is a non-tubular drape.

According to a further embodiment, the third drape includes a c-shaped clip configured to be removably secured to a portion of an arm supporting the elongated drive body, and a lower arm drape portion configured to wrap around the support arm.

According to a first aspect, a sterile barrier for a robotic drive having an elongated drive body and an articulating support arm supporting the elongated drive body is suggested, the sterile barrier comprising: a first tubular drape having a first rigid plate, a second rigid plate and a first perforated tape extending along a length of the first tubular drape, the first perforated tape being temporarily adhered to the first rigid plate and the second rigid plate to maintain a tubular shape of the first tubular drape, and the first tubular drape configured to cover a first portion of the elongated drive body when deployed.

According to an embodiment, the robotic drive includes one or more movable drive modules configured to move along a length of the elongated drive body via a channel formed along a length of a bottom wall of the elongated drive body, and removal of the first perforated tape exposes at least a portion of the channel.

According to a further embodiment. the first tubular drape includes a flexible drape body, and wherein the first rigid plate and the second rigid plate are adhered to respective edges of the flexible drape body.

According to a further embodiment, the sterile barrier further comprises: a second tubular drape having a third rigid plate, a fourth rigid plate and a second perforated tape extending along a length of the second tubular drape, the second perforated tape being temporarily adhered to the third rigid plate and the fourth rigid plate to maintain a tubular shape of the second tubular drape, and the second tubular drape configured to cover a second portion of the elongated drive body when deployed.

According to a further embodiment, the sterile barrier further comprises: a third drape configured to cover the articulating support arm, the third drape being separate from the first tubular drape and the second tubular drape.

According to a further embodiment, the third drape is a non-tubular drape.

According to a further embodiment, the third drape includes: a c-shaped clip configured to be removably secured to a portion of the articulating support arm; and a lower arm drape portion configured to wrap around the articulating support arm.

According to a further embodiment, the second tubular drape includes a flexible drape body, and wherein the third rigid plate and the fourth rigid plate are adhered to respective edges of the flexible drape body.

According to a first aspect, a method for draping a robotic drive having an elongated drive body and an articulating support arm supporting the elongated drive body is suggested, the method comprising: sliding a first tubular drape onto a first portion of the elongated drive body; sliding a second tubular drape onto a second portion of the elongated drive body, the second tubular drape being separate from the first tubular drape; and covering the articulating support arm with a third drape, the third drape being separate from the first tubular drape and the second tubular drape.

According to an embodiment, at least one of: the sliding of the first tubular drape onto the first portion includes sliding the first tubular drape onto the first portion from a first longitudinal end of the elongated drive body; or the sliding of the second tubular drape onto the second portion includes sliding the second tubular drape onto the second portion from a second longitudinal end of the elongated drive body, the second longitudinal end being opposite the first longitudinal end.

According to a further embodiment: the robotic drive includes one or more movable drive modules configured to move along a length of the elongated drive body via a channel formed along a length of a bottom wall of the elongated drive body; and the method further includes moving the one or more movable drive modules to the second longitudinal end of the elongated drive body prior to sliding the first tubular drape onto the first portion, and moving the one or more movable drive modules to the first longitudinal end of the elongated drive body prior to sliding the second tubular drape onto the second portion.

According to a further embodiment, prior to moving the one or more movable drive modules to the first longitudinal end, the method further comprises: removing a first perforated tape from the first tubular drape to expose at least a portion of the channel.

According to a further embodiment, the first tubular drape includes a first rigid plate and a second rigid plate extending along a length of the first tubular drape, and the method includes removably fixing the first rigid plate to a first side of the elongated drive body, and removably fixing the second rigid plate to a second side of the elongated drive body, the second side being adjacent the first side.

According to a further embodiment, the second tubular drape has a third rigid plate and a fourth rigid plate extending along a length of the second tubular drape, and the method includes removably fixing the third rigid plate to the first side of the elongated drive body, and removably fixing the fourth rigid plate to the second side of the elongated drive body.

According to a further embodiment, the third drape includes a c-shaped clip and a lower arm drape portion, and the covering includes removably securing the c-shaped clip to a portion of the articulating support arm, and wrapping the lower arm drape portion around the articulating support arm.

The drawings are to be regarded as being schematic representations and elements illustrated in the drawings are not necessarily shown to scale. Rather, the various elements are represented such that their function and general purpose become apparent to a person skilled in the art. Any connection or coupling between functional blocks, devices, components, or other physical or functional units shown in the drawings or described herein may also be implemented by an indirect connection or coupling. A coupling between components may also be established over a wireless connection. Functional blocks may be implemented in hardware, firmware, software, or a combination thereof.

It will be understood that, although the terms first, second, etc. may be used herein to describe various elements, components, regions, layers, and/or sections, these elements, components, regions, layers, and/or sections, should not be limited by these terms. These terms are only used to distinguish one element from another. For example, a first element could be termed a second element, and, similarly, a second element could be termed a first element, without departing from the scope of embodiments. As used herein, the term "and/or," includes any and all combinations of one or more of the associated listed items. The phrase "at least one of" has the same meaning as "and/or".

Spatially relative terms, such as "beneath," "below," "lower," "under," "above," "upper," and the like, may be used herein for ease of description to describe one element or feature's relationship to another element(s) or feature(s) as illustrated in the figures. It will be understood that the spatially relative terms are intended to encompass different orientations of the device in use or operation in addition to the orientation depicted in the figures. For example, if the device in the figures is turned over, elements described as "below," "beneath," or "under," other elements or features would then be oriented "above" the other elements or features. Thus, the example terms "below" and "under" may encompass both an orientation of above and below. The device may be otherwise oriented (rotated 90 degrees or at other orientations) and the spatially relative descriptors used herein interpreted accordingly. In addition, when an element is referred to as being "between" two elements, the element may be the only element between the two elements, or one or more other intervening elements may be present.

Spatial and functional relationships between elements (for example, between modules) are described using various terms, including "on," "connected," "engaged," "interfaced," and "coupled." Unless explicitly described as being "direct," when a relationship between first and second elements is described in the disclosure, that relationship encompasses a direct relationship where no other intervening elements are present between the first and second elements, and also an indirect relationship where one or more intervening elements are present (either spatially or functionally) between the first and second elements. In contrast, when an element is referred to as being "directly" connected, engaged, interfaced, or coupled to another element, there are no intervening elements present. Other words used to describe the relationship between elements should be interpreted in a like fashion (e.g., "between," versus "directly between," "adjacent," versus "directly adjacent," etc.).

The terminology used herein is for the purpose of describing particular embodiments only and is not intended to be limiting of the embodiments. As used herein, the singular forms "a," "an," and "the," are intended to include the plural forms as well, unless the context clearly indicates otherwise. As used herein, the terms "and/or" and "at least one of" include any and all combinations of one or more of the associated listed items. It will be further understood that the terms "comprises," "comprising," "includes," and/or "including," when used herein, specify the presence of stated features, integers, steps, operations, elements, and/or components, but do not preclude the presence or addition of one or more other features, integers, steps, operations, elements, components, and/or groups thereof. As used herein, the term "and/or" includes any and all combinations of one or more of the associated listed items. Expressions such as "at least one of," when preceding a list of elements, modify the entire list of elements and do not modify the individual elements of the list. Also, the term "example" is intended to refer to an example or illustration.

It should also be noted that in some alternative implementations, the functions/acts noted may occur out of the order noted in the figures. For example, two figures shown in succession may in fact be executed substantially concurrently or may sometimes be executed in the reverse order, depending upon the functionality/acts involved.

Unless otherwise defined, all terms (including technical and scientific terms) used herein have the same meaning as commonly understood by one of ordinary skill in the art to which embodiments belong. It will be further understood that terms, e.g., those defined in commonly used dictionaries, should be interpreted as having a meaning that is consistent with their meaning in the context of the relevant art and will not be interpreted in an idealized or overly formal sense unless expressly so defined herein.

It is noted that some embodiments may be described with reference to acts and symbolic representations of operations (e.g., in the form of flow charts, flow diagrams, data flow diagrams, structure diagrams, block diagrams, etc.) that may be implemented in conjunction with units and/or devices discussed above. Although discussed in a particular manner, a function or operation specified in a specific block may be performed differently from the flow specified in a flowchart, flow diagram, etc. For example, functions or operations illustrated as being performed serially in two consecutive blocks may actually be performed simultaneously, or in some cases be performed in reverse order. Although the flowcharts describe the operations as sequential processes, many of the operations may be performed in parallel, concurrently or simultaneously. In addition, the order of operations may be re-arranged. The processes may be terminated when their operations are completed, but may also have additional steps not included in the figure. The processes may correspond to methods, functions, procedures, subroutines, subprograms, etc.

Specific structural and functional details disclosed herein are merely representative for purposes of describing embodiments. The present invention may, however, be embodied in many alternate forms and should not be construed as limited to only the embodiments set forth herein.

## Claims

1. A draping system (50, 52, 54) for a robotic drive (24) having an elongated drive body (100) and an articulating support arm (22) supporting the elongated drive body (100), the draping system (50, 52, 54) **characterized by**:
a first tubular drape (50) configured to cover a first portion of the elongated drive body (100);
a second tubular drape (52) configured to cover a second portion of the elongated drive body (100), the second tubular drape (52) being separate from the first tubular drape (50); and
a third drape configured (54) to cover the articulating support arm (22), the third drape (54) being separate from the first tubular drape (50) and the second tubular drape (52).

2. The draping system (50, 52, 54) of claim 1, **characterized in that** the first tubular drape (50) is configured to slide onto the elongated drive body (100) from a first end of the elongated drive body (100) to cover the first portion of the elongated drive body (100).

3. The draping system (50, 52, 54) of claim 2, **characterized in that** the second tubular drape (52) is configured to slide onto the elongated drive body (100) from a second end of the elongated drive body (100) to cover the second portion of the elongated drive body (100), the second end being opposite the first end in a longitudinal direction of the elongated drive body (100).

4. The draping system (50, 52, 54) of any of the preceding claims, **characterized in that**
the first tubular drape (50) includes a first perforated tape (507) temporarily adhered along a length of the first tubular drape (50) to maintain a tubular shape of the first tubular drape (50).

5. The draping system (50, 52, 54) of claim 4, **characterized in that** the first perforated tape (507) is configured to be removed after the first tubular drape (50) is deployed on the elongated drive body (100).

6. The draping system (50, 52, 54) of claim 5, **characterized in that**
the robotic drive (24) includes one or more movable drive modules (32a-32d) configured to move along a length of the elongated drive body (100) via a channel formed along a length of a bottom wall (114) of the elongated drive body (100), and
removal of the first perforated tape (507) exposes the channel.

7. The draping system (50, 52, 54) of any of claims 4-6, **characterized in that** the first tubular drape (50) includes a first rigid plate (502) and a second rigid plate (503) extending along a length of the first tubular drape (50), and wherein the first perforated tape (507) is temporarily adhered to the first rigid plate (502) and the second rigid plate (503) to maintain the tubular shape of the first tubular drape (50).

8. The draping system (50, 52, 54) of claim 7, **characterized in that** the first tubular drape (50) includes a flexible drape body, and wherein the first rigid plate (502) and the second rigid plate (503) are adhered to respective edges of the flexible drape body (501).

9. The draping system (50, 52, 54) of any of claims 4-8, **characterized in that** the second tubular drape (52) has a third rigid plate (5212), a fourth rigid plate (5213) and a second perforated tape (5217) extending along a length of the second tubular drape (52), the second perforated tape (5217) being temporarily adhered to the third rigid plate (5212) and the fourth rigid plate (5213) to maintain a tubular shape of the second tubular drape (52).

10. The draping system (50, 52, 54) of claim 9, **characterized in that** the second tubular drape (52) includes a flexible drape body (5211), and wherein the third rigid plate (5212) and the fourth rigid plate (5213) are adhered to respective edges of the flexible drape body (5211).

11. The draping system (50, 52, 54) of any of the preceding claims, **characterized in that** the third drape (54) is a non-tubular drape.

12. The draping system (50, 52, 54) of any of the preceding claims, **characterized in that** the third drape (54) includes
a c-shaped clip (5418) configured to be removably secured to a portion of the articulating support arm (22), and
a lower arm drape portion (5419) configured to wrap around the articulating support arm (22).

13. A sterile barrier (50, 52, 54) for a robotic drive (24) having an elongated drive body (100) and an articulating support arm (22) supporting the elongated drive body (100), the sterile barrier (50, 52, 54) **characterized by**:
a first tubular drape (50) having a first rigid plate (502), a second rigid plate (503) and a first perforated tape (507) extending along a length of the first tubular drape (50), the first perforated tape (507) being temporarily adhered to the first rigid plate (502) and the second rigid plate (503) to maintain a tubular shape of the first tubular drape (50), and the first tubular drape (50) configured to cover a first portion of the elongated drive body (100) when deployed.

14. The sterile barrier (50, 52, 54) of claim 13, **characterized in that**
the robotic drive (24) includes one or more movable drive modules (32a-32d) configured to move along a length of the elongated drive body (100) via a channel formed along a length of a bottom wall (114) of the elongated drive body (100), and
removal of the first perforated tape (507) exposes at least a portion of the channel.

15. The sterile barrier (50, 52, 54) of claim 13 or 14, **characterized in that** the first tubular drape (50) includes a flexible drape body (501), and wherein the first rigid plate (502) and the second rigid plate (503) are adhered to respective edges of the flexible drape body (501).
